(19) Europäisches Patentamt
European Patent Office
Office européen des brevets

(11) Publication number: **0 147 156**
**B1**

(12) **EUROPEAN PATENT SPECIFICATION**

(45) Date of publication of patent specification: **17.08.88**

(21) Application number: **84308834.5**

(22) Date of filing: **17.12.84**

(51) Int. Cl.$^4$: **C 07 C 67/343,** C 07 C 51/353, C 07 C 69/54, C 07 C 57/04, C 07 C 67/317, C 07 C 51/377

(54) **Process for the production of alpha, beta-ethylenically unsaturated esters.**

(30) Priority: **03.01.84 US 567551**

(43) Date of publication of application:
**03.07.85 Bulletin 85/27**

(45) Publication of the grant of the patent:
**17.08.88 Bulletin 88/33**

(84) Designated Contracting States:
**AT BE DE FR GB IT NL**

(56) References cited:
**EP-A-0 055 533**
**EP-A-0 111 605**
**EP-A-0 111 998**
**DE-B-2 615 887**
**US-A-4 444 904**

(73) Proprietor: **Exxon Research and Engineering Company**
**P.O.Box 390 180 Park Avenue**
**Florham Park New Jersey 07932 (US)**

(72) Inventor: **Ryu, Ji-Yong**
**196 Nottingham Road**
**Ramsey, NJ (US)**

(74) Representative: **Dew, Melvyn John et al**
**Esso Chemical Ltd. Esso Chemical Research Centre P.O. Box 1**
**Abingdon Oxfordshire, OX13 6BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 147 156 B1

Courier Press, Leamington Spa, England.

**Description**

The present invention relates to an improved process for producing alpha, beta-ethylenically unsaturated carboxylic acid esters and free acids thereof.

It is known that olefinic compounds can be synthesized by reacting aldehydes or acetals with organic compounds containing carbonyl groups such as carboxylic acids and/or esters thereof. Such reactions can be illustrated by the following equations:

$$CH_3CH_2—CO_2—CH_3+\underset{\text{Formaldehyde}}{H_2CO}\rightarrow CH_3—\underset{\underset{CH_2}{||}}{C}—CO_2CH_3+H_2O \qquad \text{(Eq. 1)}$$

$$CH_3—CH_2—CO_2—CH_3+\underset{\text{Methylal}}{CH_2—(OCH_3)_2}\rightarrow CH_3—\underset{\underset{CH_2}{||}}{C}—CO_2—CH_3+2CH_3OH \qquad \text{(Eq. 2)}$$

It will be noted that Equations 1 and 2 use formaldehyde or methylal, respectively, as alternative reactants. In addition the methyl propionate (herein referred to also as MP) reactant can also be replaced with the free acid form, i.e. propionic acid (herein referred to also as PA).

The above reactants are conventional and each is associated with certain disadvantages, which in turn can be influenced by the choice of catalyst.

More specifically, catalysts employed for the reactions of Equations 1 or 2 can broadly be classified as predominantly basic or acidic. It is well known, that basic catalysts, when employed in conjunction with the reaction of Equation 1, will cause disproportionation of formaldehyde to $H_2$, $CO_2$, and methanol, in accordance with the Cannizzaro reaction thereby reducing the selectivity of the reaction to desired products such as methylmethacrylate (MMA) and/or methacrylic acid (MA). In addition, the use of a basic catalyst also causes decarboxylation of the co-reactant carboxylic acid or ester thereof whether formaldehyde is employed as a reactant or not, thereby further reducing the selectivity to desired products. Furthermore when formaldehyde is manufactured in the vapor phase, it is adsorbed and dissolved in water to reduce its potential to polymerize. Methanol is also employed as a polymerization inhibitor. Consequently, formaldehyde is generally sold economically as a 35—45 wt.% mixture of the same with the remainder being water and methanol and such solutions are generally employed as the formaldehyde source in Equation 1. The presence of such large amounts of water and methanol makes it difficult to economically achieve a concentrated reactant feed stream and leads to some undesirable hydrolysis of the ester product.

In view of the disadvantages of the base-catalyzed formaldehyde synthesis route, attempts have been made to replace formaldehyde with a less troublesome reactant such as dimethoxy methane, also known as methylal. However, when methylal is employed in conjunction with a base catalyst, conversion of methylal is very low. Such low conversions are believed to be attributable to the inability of the basic catalyst to efficiently hydrolyze the methylal to formaldehyde which in turn reacts with the carboxylic acid or ester co-reactant. Furthermore, when the free acid is used as a reactant (e.g. PA) methanol is usually required in excessive amounts.

A further disadvantage of the use of methylal in conjunction with such base catalyzed reactions is that methylal can react with the acid reactant (e.g. PA) to eventually form acetone and/or propylene thereby reducing the selectivity and yield to the desired products.

Many of the problems associated with basic catalysts have sought to be alleviated by the use of more active acidic catalysts. The use of such acidic catalysts however, is also associated with disadvantages. For example, the use of formaldehyde (and its associated high water content) with acidic catalysts tends to cause extensive hydrolysis of the most desired ester product, e.g. methylmethacrylate (herein also referred to as MMA), to methacrylic acid (herein also referred to as MA).

In an attempt to alleviate the hydrolysis problem associated with the use of formaldehyde (and its high water content), methylal has been used to replace formaldehyde with either the free acid or ester thereof as a co-reactant in the absence of water. Acid catalysts have also been recognized to cause decarboxylation of the ester and acid reactants as well as decomposition of formaldehyde or methylal to CO, $H_2$ and dimethyl ether at temperatures above 250°C. Thus, the selection of appropriate reactants and catalysts for conducting the subject reactions is based on a number of competing considerations to achive a desired balance in product and by-product distribution.

The present invention is based on discoveries which are believed to be applicable to acid and/or base catalyzed reactions of the type described herein whether employing acid or ester reactants with formaldehyde or methylal co-reactants.

The following discussion is intended to provide a more specific general background of the prior art for conducting the aforedescribed process.

U.S. Patent No. 3,100,795 describes the use of basic catalysts such as natural or synthetic (e.g. zeolites), alkali and alkaline earth metal aluminosilicates, as well as alkali and alkaline earth metal hydroxides supported on natural or synthetic aluminosilicates or silica gels, to catalyze the reaction between methanol, propionic acid, and formaldehyde (or methylal) to form methylmethacrylate. The use of propionic acid as

the starting material avoids the need to esterify unreacted propionic acid prior to recycle as would normally be the case when methyl propionate is employed as the reactant (see col. 1, lines 21 et. seq. and col. 2, lines 32 et. seq.). This is alleged to facilitate recycle of by-products and unreacted materials. Thus, under continuous operating conditions recovered materials (identified as consisting primarily of MMA, MP, methanol, PA, formaldehyde and water) are recycled to the starting materials mixture or feed stock, "after the methyl methacrylate has been recovered" therefrom. Thus, the recycle mixture is identified as a mixture of methanol, MP, PA, formaldehyde and no more than 3% water (col. 1, lines 61 et. seq.). The formation of dimethyl ether is not disclosed nor is the presence of the same in the recycle stream. Furthermore, since the MMA product (b.p. 101°C) is removed from the recovered materials prior to recycle (e.g. by distillation), any dimethyl ether (b.p. −24.5°C) inherently present in the product stream would be removed along with the MMA product and therefore could not be inherently present in the recycle mixture. Methanol is also employed in this reference in large excess, relative to PA, the former being alleged to be effective in preventing side reactions. The excess methanol is believed to be responsible for converting unreacted PA to MP.

U.S. Patent No. 3,014,958 is directed to a process for producing alpha, beta-ethylenically unsaturated esters wherein a substantial amount of ester product is introduced into the feed stream, containing formaldehyde and reactant ester (e.g. MP), which is contacted with acid or base catalysts. This patent fails to disclose the formation of dimethyl ether during reaction or recycle of the same to the feed stream.

U.S. Patent No. 3,840,588, assigned to Monsanto, describes the use of alkali metal hydoxides or oxides dispersed on a support having a surface area of 350 to 1000 $m^2$/gm. Suitable support materials include aluminas, thorias, magnesias, silica-aluminas, and silicates. In addition to hydroxides or oxides, other alkali metal compounds may be deposited on the support such as carbonates, nitrates, sulphates, phosphates, inorganic salts, acetates, propionates or other carboxylates. All of such supported catalysts are basic catalysts. These catalysts are employed in the reaction of formaldehyde and saturated alkyl carboxylates to form alpha, beta-ethylenically unsaturated esters at temperatures of at least 400 to 600°C. A methylmethacrylate selectivity of 82 mole % at formaldehyde conversions of 98% are reported in this patent at a reaction temperature of 400°C and a space time yield of 490 l/hr (Table II, Run 7). However, at 430°C and higher space time yields of 960 l/hr (Example 1) the selectivity to methylmethacrylate of 92 mole % is obtained at a formaldehyde conversion of only 67%. At reaction temperatures below 400°C, it is alleged that selectivities drop significantly, e.g., to below 40% (see Fig. 2) due to the Cannizzaro reaction (col. 3, lines 29 et seq.). Moreover, water must be employed in the feed stream in strictly controlled amounts to obtain good selectivity. In the absence of water, formaldehyde conversion is negligible, and in the presence of too much water selectivity drops drastically. The required use of water necessitates the use of alcohols in the feed stream to suppress hydrolysis of the ester reactant and reduce the amount of ester in the reaction zone by acting as a diluent (see col. 3, lines 55 et. seq.) as well as complicating the overall process to implement strict control of the water content of the feed stream. This patent does not disclose the formation of dimethyl ether or recycle of the same to the feed stream.

U.S. Patent No. 3,933,888, assigned to Rohm and Haas Co., discloses the reaction of formaldehyde with an alkanoic acid or its ester in the presence of basic catalysts containing basic pyrogenic silica (e.g. SA of 150 to 300 $m^2$/g) alone or impregnated with activating agents which provide additional basic sites to the pyrogenic silica when callcined. Such activating agents include alkali and alkaline earth metal hydroxides, oxides, amides, and salts such as carbonates, oxalates, phosphates, e.g., $Na_3PO_4$, $Na_2HPO_4$, $KOCH_3$, $Na_4SiO_4$. The identity, impregnation and calcination procedures of the activating agent is always selected to provide a basic catalyst. A molar ratio of alkanoic acid:formaldehyde:water:methanol of from 1:1:0.01:0 to 1:1:6:0.03 is disclosed. In Example 2, which employs a molar ratio of propionic acid:formaldehyde:water:methanol of 20:20:59:1, conversion of formaldehyde and propionic acid to methacrylic acid and methylmethacrylate is 34% and selectivity (referred to in this patent as yield) to MA+MMA based on propionic acid converted is 71%. Howver, in Example 24, which replaces propionic acid with methyl propionate at the same respective molar ratio, the selectivity to MA+MMA, based on a propionic acid conversion of 25 drops to 44%. Furthermore, from the data of Table III in this patent, it can be calculated that for every 100 moles of formaldehyde in the feed, 34 moles thereof are converted to MA+MMA and 45 moles thereof remain unreacted. About 21 moles of formaldehyde are unaccounted for. This patent fails to disclose the formation of dimethyl ether or recycle of the same to the feedstream.

U.S. Patent No. 4,118,588 assigned to BASF, is directed to a process for synthesizing alpha, beta-ethylenically unsaturated acids or esters such as methacrylic acid and methylmethacrylate from the reaction of propionic acid and/or methylpropionate with dimethoxymethane (methylal) in the presence of catalysts (most of which are acidic) based on one or more salts selected from phosphates and silicates of: magnesium, calcium, aluminum, zirconium, thorium and titanium. Such salts can be used alone or together with oxides of the same aforedescribed magnesium et al metals and additionally boric acid and/or urea. Thus, a typical acidic catalyst consists of aluminum phosphates, titanium dioxide, boric acid and urea. Included within the list of 62 possible combinations of various materials are aluminum phosphate and aluminum silicate or aluminum phosphate, aluminum silicate and boric acid. Such catalysts can be modified with alkali and/or alkaline earth metal: carboxylates, oxides, silicates and hydroxides. The above catalysts are employed in a reaction system which contains 0 to 0.5 mole $H_2O$ per mole of methylal. Methyl propionate is preferably employed in a large molar excess relative to methylal (e.g. 2:1 to 6:1). It is alleged

that since water is not deliberately introduced nor produced by the reaction, hydrolysis of the esters is avoided and it is therefore not necessary to use methanol as a starting material. It is not seen how the formation of water can be avoided, although the use of excess MP would consume excess $H_2O$ produced in situ. This patent also fails to disclose the formation of dimethyl ether or recycle of the same to the feed stream.

U.S. Patent No. 4,147,718, assigned to Rohm GmbH, is directed to a method for making alpha, beta-unsaturated carboxylic acids and their functional derivatives, such as methacrylic acid and methylmethacrylate, from the reaction of methylal (dimethoxymethane) with propionic acid or its corresponding ester or nitrile, in the presence of a catalyst, which catalyst is a combination of silicon dioxide provided with basic sites (as described in U.S. Patent No. 3,933,888) and aluminum oxide, which optionally may also be provided with basic sites in a similar manner. When propionic acid is employed as the reactant, at least an equivalent amount of alcohol is also employed. The formation of dimethyl ether is not disclosed nor is recycle of the same.

U.S. Patent 4,324,908, assigned to SOHIO, is directed to a promoted phosphate catalyst for use in synthesizing alpha, beta-unsaturated products, which catalyst requires the presence of at least one or more of Fe, Ni, Co, Mn, Cu, or Ag, as promoters in conjunction with phosphorus and oxygen. No mention is made of the formation of dimethyl ether or recycle of the same.

Albanesi, G., and Moggi, P., Chem. Ind. (Milan) Vol. 63, p. 572—4 (1981) disclose the use of Groups 3, 4 and 5 metal oxides in unsupported or $SiO_2$ supported form, for the condensation reaction between the methyl hemiacetal of formaldehyde ($CH_3OCH_2OH$) and methylpropionate to form methylmethacrylates. Ten percent $WO_3$ supported on $SiO_2$ is reported as the best catalyst relative to other disclosed catalysts because the decomposition of formaldehyde to CO and $H_2$ and the decarboxylation of methylpropionate, occur least over this catalyst. However, the highest reported formaldehyde conversion when employing the tungsten catalyst is only 37.5%. Furthermore, it is disclosed that gamma-alumina, silica-alumina and molecular sieves tend to convert the hemiacetal of formaldehyde to dimethylether at 250°C, while at higher temperatures (e.g., above 400°C) formaldehyde is decomposed to CO and $H_2$. Thus, this reference recognizes the formation of dimethyl ether, but refers to the same as a non-utilizable by-product.

In view of the above, it can be seen that very little attention has been given to the formation of dimethylether during the aforedescribed condensation reactions and when it has, dimethylether has been considered an undesirable by-product.

Accordingly, because of the commercial importance of alpha, beta-unsaturated products, such as methylmethacrylate, there has been a continuing search for ways to maximize the use of by-products as process credits by either converting them to desirable, commercially disposable products or recycling them for continued advantageous use in an overall process scheme. The present invention is a result of this search.

## Summary of the invention

The essence of the present invention resides in the advantageous use of ether by-products, such as dimethyl ether (DME), previously considered undesirable and non-utilizable in alpha, beta-ethylenically unsaturated ester producing condensation reactions. More specifically, such ether by-products (e.g., DME) are employed in the present invention for several purposes, namely, (1) to convert the acid reactant, e.g., propionic acid, to its corresponding ester, e.g. methyl propionate, (2) to act as a water scavenging agent and (3) to convert unsaturated acid product, e.g., methacrylic acid, to its corresponding ester, e.g., methylmethacrylate. The advantages obtained by employing such ether by-products for these purposes stems from the fact that the combined selectivity to the unsaturated ester and acid products, e.g., MMA and MA is increased when the reactant is an ester, e.g., MP, rather than its corresponding acid, e.g., PA, and that the unsaturated acid product, e.g., MA, is decomposed in higher amounts when contacted with a solid (e.g., acidic) dehydration catalyst in the presence of water relative to the extent of decomposition of the corresponding unsaturated ester product, e.g., MMA, in contact with said catalyst under comparable reaction conditions. Therefore, it has been found desirable to enhance selectivity of the reaction to the unsaturated ester, e.g., MMA, at the expense of its corresponding acid, e.g., MA, in the product stream and enhance the concentration of the ester reactant, e.g., MP, in the feed stream at the expense of its corresponding acid, e.g., PA.

In-situ sources of the ether by-products include:

(a) the decomposition of methylal as illustrated by the following reaction:

$$CH_2(OCH_3)_2 \rightarrow CH_2O + CH_3OCH_3 \quad \text{(Eq. 3)}$$

(b) hydrolysis of the ester reactant to alcohol and dehydration of the alcohol to dimethyl ether as illustrated by the following reactions using methyl propionate as the ester reactant:

$$CH_3CH_2CO_2CH_3 + H_2O \rightleftharpoons CH_3CH_2CO_2H + CH_3OH \quad \text{(Eq. 4)}$$

$$2CH_3OH \rightleftharpoons CH_3OCH_3 + H_2O \quad \text{(Eq. 5)}$$

(c) decarboxylation of the ester reactant in accordance with the following illustrative reaction using methyl propionate as an example:

$$2CH_3CH_2CO_2CH_3 \rightarrow CH_3—CH_2—\overset{\overset{\displaystyle O}{\|}}{C}—CH_2—CH_3+CH_3OCH_3+CO_2 \qquad \text{(Eq. 6)}$$

and

(d) the reaction of methylal with the ester or acid reactant as illustrated by the following reactions using propionic acid and methylpropionate as examples:

$$CH_2(OCH_3)_2+CH_3—CH_2—CO_2H \rightarrow CH_3—\overset{\overset{\displaystyle CH_2}{\|}}{C}—CO_2H+CH_3—O—CH_3+H_2O \qquad \text{(Eq. 7)}$$

$$CH_2(OCH_3)_2+CH_3—CH_2—CO_2CH_3 \rightarrow CH_3—O—CH_3+CH_3—\overset{\overset{\displaystyle CH_2}{\|}}{C}—CO_2CH_3+H_2O \qquad \text{(Eq. 8)}$$

The functions of the ether by-product as employed in accordance with the present invention can be illustrated by the following representative reactions:

$$\underset{\text{(PA)}}{CH_3CH_2CO_2H}+\underset{\text{(DME)}}{CH_3OCH_3} \rightleftharpoons \underset{\text{(MP)}}{CH_3CH_2CO_2CH_3}+CH_3OH \qquad \text{(Eq. 9)}$$

$$\underset{\text{(MA)}}{CH_3—\overset{\overset{\displaystyle CH_2}{\|}}{C}—CO_2H}+\underset{\text{(DME)}}{CH_3OCH_3} \rightleftharpoons \underset{\text{(MP)}}{CH_3—\overset{\overset{\displaystyle CH_2}{\|}}{C}—CO_2CH_3}+CH_3OH \qquad \text{(Eq. 10)}$$

$$\underset{\text{(DME)}}{CH_3OCH_3}+H_2O \rightleftharpoons 2CH_3OH \qquad \text{(Eq. 11)}$$

Regarding Equation 9, the acid reactant, e.g. propionic acid, is more susceptible to undesired side reactions than its corresponding ester, e.g. methyl propionate, such as decarboxylation and the formation of ketones, as well as reaction with methylal or formaldehyde to produce an undesired product distribution containing acetone, $CO_2$, acrylic acid and propylene. Thus, by employing the ether DME to convert the acid reactant to its ester form, such undesired side reactions are reduced and selectivity to methylmethacrylate is enhanced. Furthermore, since the unsaturated ester product has been found to be more stable than its corresponding acid form, in terms of forming non-utilizable by-products, the enhanced selectivity to methyl methacrylate actually results in an increase in the combined MMA+MA selectivity. The stability of the unsaturated ester product is further utilized by employing an ether, e.g. DME, to directly convert in-situ the unsaturated acid product, (e.g. MA) which is formed, to its ester form (e.g. MMA).

Furthermore, in addition to the in-situ water formation illustrated by Equations 1 and 2 above, the presence of methanol in the reaction mixture, whether from intentional addition or from in-situ formation, can also generate additional water by reaction with the acid reactant (e.g. PA) or the unsaturated acid product. The presence of water (particularly when employing acid catalysts) in the feed and product effluent can lead to undesirable hydrolysis of the ester reactant and product. Consequently, an ether, e.g. DME is also advantageously employed as means for shifting the equilibrium reaction of Equation 11 to scavenge undesirable water from the reaction system.

Accordingly, in one aspect of the present invention there is provided an improved process for reacting a vaporous mixture comprising (a) at least one member selected from the group consisting of saturated carboxylic acid, and the ester of said carboxylic acid and (b) at least one member selected from the group consisting of formaldehyde and a derivative of formaldehyde, in the presence of a dehydration catalyst to form, by a condensation reaction, a product comprising at least one member selected from the group consisting of alpha, beta-ethylenically unsaturated acid and the ester of said unsaturated acid. The improvement comprises introducing at least one ether into said vaporous mixture in a manner and under conditions sufficient to cause an increase in the proportional distribution of said alpha, beta-ethylenically unsaturated acid ester in said product relative to the absence of said ether introduction; said ether being characterized by the ability to (a) convert said carboxylic acid reactant to its corresponding ester by reaction therewith; (b) convert said alpha, beta-ethylenically unsaturated acid product to its corresponding ester by reaction therewith; and optionally, (c) hydrolyze under acidic reaction conditions in the presence of water to its corresponding alcohol.

Brief description of the drawings

Figure 1 is a schematic representation of an embodiment of the process of the present invention wherein propionic acid, formaldehyde and methanol are introduced to a condensation reactor and recycle of DME, MP, PA and methanol is employed.

Figure 2 is a schematic representation of an embodiment of the process of the present invention wherein PA and methylal are introduced into a condensation reactor along with recycled DME, MP, PA and unreacted methylal and/or methylal derived formaldehyde.

Figure 3 is a schematic representation of an embodiment of the process of the present invention wherein an esterification reactor is employed to convert reactant and product acids to esters with DME and the resulting esterified reactants condensed with methylal along with recycled DME, MP and methylal in a condensation reactor.

Figure 4 is a schematic representation of an embodiment of the process of the present invention wherein an esterification reactor is employed to convert recycled PA and MA to esters with DME and condensing MP with methylal in the presence of recycled DME, methylal and MP.

Description of preferred embodiments

The present invention is directed to an improvement in well-known processes for synthesizing alpha, beta-ethylenically unsaturated esters. Such esters are typically produced as a mixture of the ester and the free acid of the ester and it is the proportional distribution of these two products which is sought to be controlled by the use of a heretofore little recognized and seemingly non-utilizable by-product of such reactions.

The starting reactant materials are well known and comprise saturated carboxylates, preferably monocarboxylates and acids thereof and formaldehyde or derivatives thereof. Suitable saturated carboxylates and acid derivatives can be represented by the structural formula:

$$R—CH_2—COOR' \qquad (I)$$

wherein R and R' are independently selected from the group consisting of hydrogen and a hydrocarbyl group selected from alkyl, typically $C_1$ to $C_{20}$, preferably $C_1$ to $C_{10}$ alkyl and most preferably $C_1$ to $C_5$ alkyl; aryl, typically $C_6$ to $C_{14}$ aryl, preferably $C_6$ to $C_{10}$ aryl, and most preferably $C_6$ aryl; aralkyl and alkaryl wherein the alkyl and aryl portions thereof are as described immediately above. Preferably R is other than hydrogen. Most preferably R and R' are alkyl.

It will be understood that as to the particular selection of R of structural formula I, no limitation on the operability is to be expected, except where R would itself be reactive or where R would present steric hindrance rendering the reaction difficult as would be obvious to the skilled artisan. Similar considerations apply with respect to R'. From a practical standpoint, the identity of R and R' is typically selected to permit the corresponding unsaturated product to be readilya vaporizable without substantial decomposition.

Representative carboxylates suitable for use in this invention include methyl acetate, ethyl acetate, methyl propionate, ethyl propionate, methyl n-butyrate, the methyl ester of phenyl acetic acid, as well as the corresponding free acids thereof.

The most preferred compounds represented by structural formula I are propionic acid, methyl propionate and mixtures thereof.

The co-reactant is formaldehyde or suitable derivatives thereof, including polymers, oligomers and precursors of formaldehyde. Thus commercial aqueous or alcoholic $CH_2O$ solutions, formals such as methylal or volatile $(CH_2O)_x$ compounds, such as s-trioxane, as well as the hemiacetal of formaldehyde (i.e. $CH_2OHOCH_3$), may serve as a formaldehyde source. Alternately, the gaseous product effluent from a formaldehyde manufacturing process unit (e.g. by methanol oxidation) may be passed over a catalyst along with the vapors of the appropriate ester or acid. Alternatively, vaporous formaldehyde effluent adsorbed in methanol, or methanol/MP mixtures can be employed as the formaldehyde source.

The preferred formaldehyde source is methylal since this material enables one to avoid the use of excess dimethyl ether to scavenge excess water (normally associated with the use of formaldehyde directly) and reduce hydrolysis of the ester reactant and product. A formaldehyde/methanol solution formed by adsorbing formaldehyde vapor (resulting from a methanol oxidation reactor) with methanol is an alternative preferred formaldehyde source, due to the low water content associated therewith.

The identity of the ether by-product which is recycled in accordance with the process of the present invention will depend on the nature of the reactants employed. Thus, if methylal is employed, the ether by-product will comprise dimethyl ether. In addition, ethers represented by the structural formula:

$$R'—O—R' \qquad (II)$$

wherein R' is as described in conjunction with, and derived from the compounds represented by structural formula I above, will be produced in-situ and further comprise the recyclable ether by-products.

Representative ethers include dimethyl ether, diethylether, dipropylether, diphenyl ether, methylethylether, dibenzyl ether. The most preferred ether by-product for recycle is dimethyl ether.

While the process of the present invention finds its most practical application in the recycle of ether

by-products, any ether capable of performing the aforenoted functions can be supplied solely from an independent source or in combination with recycled by-product ether.

As would be obvious to the skilled artisan, the resulting alpha, beta-ethylenically unsaturated product derived from reactants represented by structural formula (I), can be represented by the structural formula:

$$\begin{array}{l} R{-}C{-}COOR' \\ \quad\ \| \\ \quad CH_2 \end{array} \qquad (IIa)$$

wherein R and R' are as described in conjunction with structural formula (I).

Although not preferred, diluents such as water (to reduce coking of conventional catalysts) and lower organic alcohols, typically those which are produced upon hydrolysis of the ester reactant or product can be included in the feedstream.

The dehydration catalysts which can be employed include any of the conventional solid dehydration catalysts described in the background patents above.

Representative acidic catalysts include aluminum oxide, aluminum oxide/iron oxide/titanium oxide (Br. Patent Specification No. 1,491,183); zirconium oxide, zirconium oxide/aluminum oxide, zirconium oxide/silicon oxide, zirconium oxide/aluminum oxide/silicon oxide (Br. Patent Specification No. 2,001,647B); aluminum phosphate (U.S. Patent No. 3,014,958); titanium oxide/calcium phosphate/boric acid (U.S. Patent No. 4,118,588); acidic salts on silica such as potassium dihydrogen phosphate/$SiO_2$ (U.S. Patent No. 3,014,958). Representative basic catalysts include potassium hydroxide on silica gel (U.S. Patent No. 3,840,588); cesium hydroxide on silica gel (U.S. Patent No. 3,933,888); lithium hydroxide on aluminum phosphate (U.S. Patent No. 4,118,588); potassium oxide/magnesium oxide/iron oxide (U.S. Patent No. 3,014,958); and the metal oxides described in Albanesi et al article discussed hereinabove. All of the references discussed hereinabove are incorporated herein by reference.

The preferred catalysts, however, are acidic catalysts, the most preferred acidic catalysts being described in commonly assigned U.S. Patent 4,444,904.

More specifically, the most preferred catalyst for use in the present invention comprises an inorganic amorphous or substantially amorphous acidic oxide material comprising the following components reacted therein:

$$M^1/M^2/P/O \qquad (III)$$

wherein $M^1$ is at least one Group 3b element (of the Periodic Chart) selected from Al, Ga, In and Tl, preferably aluminum, $M^2$ is at least one Group 4b or 4a element selected from Si, Sn, Ge and Zr, preferably Si. For ease of discussion and description the aforedescribed Group 3b and 4b elements constituting $M^1$ and $M^2$ are referred to generically as metals, although it is recognized that the term "metal" as applied to Si is an unconventional use of this term. Such a catalyst is described in EP—A—111998.

It is to be understood that the precise structure of the metal-phosphorus oxide catalysts described hereinafter has not yet been determined although the components of the catalyst composition are believed to be reacted with each other during the preparative procedure and the resulting catalyst is therefore not believed to be a mere mixture of oxides.

Such catalyst compositions of formula III are prepared by reacting at least one Metal ($M^1$) Hydrocarboxide (referred to herein as Hydrocarboxide I), at least one Metal ($M^2$) Hydrocarboxide (referred to herein as Hydrocarboxide II), at least one acidic phosphorus-oxygen containing compound, and water in the presence of at least one liquid organic medium under conditions and in a manner sufficient to form a catalyst precursor composition which is then calcined to form an acidic catalyst composition.

Hydrocarboxides I and II are selected to be capable of undergoing hydrolysis of the organic portion thereof in the presence of water, and capable of being solubilized or at least partially solubilized in the organic medium and other components of the reaction mixture.

Suitable Hydrocarboxides I which can be employed as the starting material can be represented by the structural formula:

$$(M^1){-}(OR'')_3 \qquad (IV)$$

wherein $M^1$ is as described above, preferably Al, and R" is a substituted or unsubstituted hydrocarbyl radical independently selected from the group consisting of alkyl, typically alkyl having from about 1 to about 8 carbons, preferably from about 2 to about 6 carbons, and most preferably from about 3 to about 4 carbons, aryl, typically aryl having from 6 to about 14 carbons, preferably from about 6 to about 10 carbons, and most preferably 6 carbons, aralkyl and alkaryl, typically aralkyl and alkaryl wherein the alkyl and aryl portions thereof are as defined immediately above respectively; cycloalkyl, typically cycloalkyl having from about 4 to about 12 carbons, preferably from about 5 to about 10 carbons, and most preferably from about 6 to about 8 carbons, all of the above described hydrocarbyl carbon numbers being exclusive of substituents; said R" substituents being also selected from ether groups, typically ether groups represented by the structural formulae: $-O-R_1$, $-R_1-O-R_2$, wherein $R_1$ and $R_2$ are independently selected from the

group consisting of alkyl, typically about $C_1$ to about $C_{10}$ alkyl, preferably about $C_1$ to about $C_5$ alkyl, and most preferably about $C_1$ to about $C_3$ alkyl; and ester groups, typically ester groups represented by the structural formulae

$$-\overset{O}{\overset{\|}{C}}-O-R_1,\quad -O-\overset{O}{\overset{\|}{C}}-R_1,\quad -R_2-O-\overset{O}{\overset{\|}{C}}-R_1,\quad \text{and}\quad R_2-O-\overset{O}{\overset{\|}{C}}-R_1-,$$

wherein $R_1$ and $R_2$ are as defined above.

Preferred Hydrocarboxide I compounds include the alkoxides.

Representative examples of suitable Hydrocarboxides I of formula II include: aluminum tri: n-butoxide, sec-butoxide, isobutoxide, isopropoxide, n-propoxide, ethoxide, methoxide, phenoxide, benzoxide, naphthoxide, methoxyethoxide, 3-(methoxy carbonyl) propoxide, 3-(ethylcarbonyloxy)-butoxide, cyclohexoxide, 1,3-(dimethyl)-2-phenoxide, 1,2-(methoxy)-4-benzoxide, and mixtures thereof.

Similar representative hydrocarboxides can be formed replacing part, or all of the aluminum present therein with any one or more of the other aforedescribed Group 3b elements.

The preferred Hydrocarboxides I include aluminum: sec-butoxide, n-butoxide, n-propoxide, isopropoxide, methoxide, ethoxide; and mixtures thereof.

Hydrocarboxide II which is employed as a starting material in the precursor forming reaction can be represented by the structural formula:

$$(M^2)-(OR'')_4 \qquad (V)$$

wherein $M^2$ and R″ are as described above in connection with structural formulae (II) and (III) above, respectively. The specific hydrocarboxide R″ groups can be the same as illustrated above in connection with the aluminum hydrocarboxides and can be employed with any of the aforedescribed Group 4b or 4a elements.

Preferred Hydrocarboxides II include silicon: tetraethoxide, tetra-n-propoxide, tetraisopropoxide, tetramethoxide, tetra-n-butoxide, tetraisobutoxide, and mixtures thereof.

The acidic phosphorus-oxygen containing compound of formula III which can be employed as a starting material for the preferred catalysts of this invention must possess at least one acidic hydrogen and be capable of reacting with the Hydrocarboxides I and II or the hydrolyzed inorganic product thereof, and the use of the term "acidic phosphorus oxygen compound" is indicative of this requirement. Representative examples of suitable acidic phosphorus-oxygen containing compounds include phosphorus acid ($P(OH)_3$), phosphorus acid ($HP(OH)_2$), phosphinous acid ($H_2POH$), phosphenous acid ($O{=}POH$), phosphoric acid ($P(O)(OH)_3$), phosphonic acid ($HP(O)(OH)_2$), phosphinic acid ($H_2P(O)(OH)$), phosphenic acid ($O{=}P(O)OH$), phosphine oxide ($H_3PO$), phosphoranoic acid ($H_4POH$), phosphorane dioic acid ($H_3P(OH)_2$), phosphorane trioic acid ($H_2P(OH)_3$), phosphoranetetroic acid ($HP(OH)_4$), phosphorane pentoic acid ($(P)(OH)_5$), as well as any of the aforenoted acids having one or more but not all of the acidic hydrogens replaced with an alkyl group, typically $C_1$ to $C_{10}$, preferably $C_1$ to $C_5$ and most preferably $C_1$ to $C_3$ alkyl.

In addition, polyphosphoric acid, an acid commercially available as a mixture of orthophosphoric acid with pyrophosphoric, triphosphoric and higher acids, sold on the basis of its calculated $H_3PO_4$ content (e.g. 115%), and super phosphoric acid sold at 105% $H_3PO_4$ content, can also be employed as starting materials.

The preferred acidic phosphorus-oxygen compound is phosphoric acid.

Upon hydrolysis of Hydrocarboxides I and II and reaction with the acidic phosphorus oxygen compound, organic alcohols are formed. Since it is desired that residual organic material in the catalyst composition be minimized, it is preferred as a matter of convenience to select the identity of the organic moiety of the Hydrocarboxides such that the alcohols derived therefrom can be easily vaporized, e.g., alkoxides having fewer than about 10 carbons are most preferred.

The organic medium used in the preparation of the catalyst precursor to the formula III compounds should be a liquid at reaction temperature and is selected from: aldehydes, ketones, ethers, and mixtures thereof typically containing from about 1 to about 20, preferably from about 1 to 10, and most preferably 1 to 5 carbon atoms.

More specifically, the organic moiety to which the aldehyde, ketone, and ether functional groups can be attached includes alkyl, typically about $C_1$ to $C_{20}$, preferably about $C_1$ to $C_{10}$, most preferably about $C_1$ to $C_5$ alkyl, aryl, typically about $C_6$ to $C_{14}$, preferably about $C_6$ to $C_{10}$, most preferably $C_6$ aryl, cycloalkyl, typically about $C_4$ to $C_{20}$, preferably about $C_6$ to $C_{12}$, most preferably about $C_6$ to $C_{10}$ cycloalkyl, aralkyl and alkaryl wherein the alkyl and aryl groups thereof are described above.

Each class of liquid organic medium can contain one or more, typically 1 to 3, functional groups as well as mixtures of functional groups. Furthermore, the preferred organic moiety of liquid organic medium is a saturated aliphatic compound.

Representative aldehydes include benzaldehyde, acetaldehyde, propionaldehyde, m-tolualdehyde, trioxane, valeraldehyde, butyraldehyde, oxalaldehyde, malonaldehyde, adipaldehyde.

Representative ketones include acetone, 3-pentanone, methylethylketone, cyclohexanone, dimethyl

ketone, diethyl ketone, dibutyl ketone, methyl isopropyl ketone, methyl sec-butyl ketone, benzophenone, and mixtures thereof.

Representative ethers include dimethyl ether, diethyl ether, dibutyl ether, tetrahydrofuran, anisole, dioctyl ether, 1,2-dimethoxyethane, 1,4-dimethoxybutane, diethylene ether, 1,1,3,3-tetramethoxypropane, and mixtures thereof.

Preferred organic media comprise acetone, diethylether, acetaldehyde, methylethyl ketone, 3-pentanone, 1,2-dimethoxyethane and mixtures thereof.

The most preferred organic medium is acetone or a mixture of acetone and diethylether.

The organic medium is believed to undergo electrostatic field interactions with the metals of Hydrocarboxides I and II and the reaction intermediates which form upon contact of the Hydrocarboxides I and II, the acidic phosphorus compound and water in the reaction mixture. This interaction is believed to occur through complexation of the organic medium with the various species present in the reaction mixture. Thus, the organic medium is not inert and only certain organic media have been found suitable for this purpose as described herein. The organic medium also functions as a solvent and/or suspending agent for the Hydrocarboxides I and II and phosphorus containing compound, and any complexes and reaction intermediates thereof, as a solvent and/or suspending agent of the resulting catalyst precursor, as a liquid for providing uniform heating and/or mixing of the catalyst forming reactants, and as a medium capable of bringing Hydrocarboxides I and II, the phosphorus-oxygen compound, and water into intimate contact for reaction. To perform the latter function it is desirable to select the organic medium such that it is at least miscible, preferably soluble, with or in, water, the catalyst forming reactants, and the Hydrocarboxide derived alcohol. It is also preferred to select the organic medium so that it will be completely or substantially removed from the catalyst precursor during drying and/or calcination. Thus, organic media with low molecular weight, and high vapor pressure are preferred. Minor amounts of alcohol, such as the hydrocarboxide derived alcohol can be tolerated within the organic medium initially or as it forms. Minor amounts of esters can also be included although this is not preferred. By minor amount as used herein is meant less than 50%, preferably less than 25%, and most preferably less than 10%, by weight of the organic medium. Minor amounts of inert diluents can be employed to reduce the cost of organic medium, such as paraffins, aromatic compounds, and mixtures thereof, although this is not preferred.

Thus, the organic medium is selected so that it is a liquid at reaction temperature, preferably dissolves, or at least partially dissolves the precursor forming reactants and comprises at least 50%, preferably at least 75%, and most preferably at least 90% (e.g. 100%), by weight thereof, of any one or more of said aldehyde, ketone, and ether. It is preferred to exclude the presence of any organic alcohol, ester, or acid from the initial starting composition of the liquid organic medium.

The catalyst precursor forming reaction for the compounds of formula III is conducted by providing a reaction admixture comprising at least one Hydrocarboxide I, at least one Hydrocarboxide II, water, and liquid organic medium. However, the order of addition of the components is critical to the extent that it must be conducted in a manner sufficient to avoid contact of either of the Hydrocarboxides I and II with water prior to contact of said Hydrocarboxides I and II with the acidic phosphorus-oxygen containing compound, to avoid premature reaction of the water and the Hydrocarboxides I and II. Thus, a wide variety of admixture sequences are possible subject to the above constraints.

The preferred method of admixture is to initially prepare two separate solutions typically at ambient temperature and pressure. The first solution contains Hydrocarboxides I and II dissolved in a suitable organic liquid medium. The second solution contains the acidic phosphorus oxygen compound, water, and organic liquid medium, preferably the same medium used in, or at least miscible with, the first solution. The two solutions are then mixed, preferably by the addition of Solution 2 to Solution 1. While very small amounts of water may be tolerated in the first solution, it is preferred that it be anhydrous. An alternative preferred variation is to withhold a portion of the needed amount of Hydrocarboxides I and/or II from the first solution (e.g. withhold about 30% by weight of the total Hydrocarboxide I and/or II, combine the solutions and then add the remainder of Hydrocarboxide I and/or II. Stepwise addition of the Hydrocarboxides can also be accompanied with stepwise addition of organic medium. An alternative addition procedure is to prepare 3 separate solutions containing respectively, Hydrocarboxide I and liquid organic medium (Solution 1), Hydrocarboxide II and liquid organic medium (Solution 2), and the acid phosphorus oxygen compound, water, and liquid organic medium (Solution 3). The solutions are then combined simultaneously or individually by separately adding Solution 3 to Solution 1 and/or 2, and admixing the resulting solutions.

The relative amounts of Hydrocarboxides I and II and acidic phosphorus-oxygen containing compound employed to form the catalyst precursor forming admixture determines the gram atom ratios of the components in the catalyst. Thus, while any effective amount of said materials may be initially present in said admixture, it is contemplated that such effective amounts constitute a mole ratio of Hydrocarboxide I: Hydrocarboxide II of typically from about 1:3.5 to about 1:0.5, preferably from about 1:2 to about 1:0.7, and most preferably from about 1:1.5 to about 1:0.8. The mole ratio of Hydrocarboxide I: acidic phosphorus-oxygen compound in the reaction mixture is typically controlled to be from about 1:1.5 to about 1:0.5, preferably from about 1:1.25 to about 1:0.7, and most preferably from about 1:1.1 to about 1:0.85.

Water is also critical to the catalyst preparative process for synthesizing the compounds of formula III. The water hydrolyzes Hydrocarboxides I and II to form alcohols and corresponding metal oxides and/or

hydroxides. Consequently, the amount of water employed is related to the amount of Hydrocarboxides I and II present in the reaction admixture and preferably is effective to obtain complete hydrolysis thereof. Exact stoichiometric ratios, however, are not required. Thus, while any effective amount of water can be employed to form the reaction admixture, it is contemplated that such effective amounts constitute a mole ratio of the sum of the moles of Hydrocarboxides I and II: $H_2O$ of typically from about 3:1 to about 1:300, preferably from about 2:1 to about 1:10, and most preferably from about 1:1 to about 1:6.

The precursor forming reaction in the synthesis of the components of formula III must be conducted in the presence of at least some liquid organic medium (the composition of which is defined above). As the amount of suitable ether, aldehyde, and/or ketone liquid organic medium employed in the reaction mixture is decreased, the concentration of the Hydrocarboxide derived alcohol produced in-situ increases to the extent that the aforedescribed complexation is decreased and the undesirable effects associated with employing alcohol as the predominant organic medium during the precursor formation become increasingly more pronounced, namely, the yield of the alpha, beta-unsaturated products described herein suffers. The amount of organic medium present during the precursor forming reaction is therefore selected to effect a stirrable solution or partial solution of reactants, and improve the yield of alpha, beta-unsaturated product derived from the use of the resulting catalyst relative to the yield of said product obtainable from a catalyst prepared in the absence of said organic medium. Thus, while any effective amount of organic medium may be employed, it is contemplated that such effective amount constitute typically at least about 25%, preferably at least about 40%, and most preferably at least about 50%, and can range typically from about 25 to about 95%, preferably from about 40 to about 90%, and most preferably from about 60 to about 85%, by weight, of the reaction admixture, based on the combined weight of Hydrocarboxides I and II, the phosphorus-oxygen compound, organic medium and water.

Furthermore, it is contemplated that the amount of water in the reaction mixture used to synthesize the compounds of formula III is controlled to be typically not greater than about 25%, preferably not greater than about 20%, and most preferably not greater than about 15%, and will vary typically from about 5 to about 25%, preferably from about 8 to about 20%, and most preferably from about 10 to about 15%, by weight, based on the combined weight of liquid organic medium and water in the precursor forming admixture.

The resulting admixture is preferably mixed vigorously and continuously during its formation and during the reaction to effect intimate contact and reaction between the component reactants of the admixture. This can be achieved with conventional stirring means, by refluxing or both. Thus, in a batch operation an especially convenient means of conducting the admixing is to mechanically stir one solution while admixing into it the other solution. In a continuous mixing operation a convenient means of conducting the admixing is to simultaneously pump the two solutions through a single means such as an in-line mixer. If refluxing is employed during the catalyst precursor forming reaction, the liquid organic medium is preferably selected so that it will boil at the selected reaction temperatures described hereinbelow. Removal of the Hydrocarboxides I and II derived alcohol by-product by distillation can also be employed and is preferred when large amounts of said alcohol by-product are produced in-situ.

The formula III compound precursor forming reaction temperature is effective to achieve complete reaction and is controlled in conjunction with the pressure and in a manner sufficient to avoid vaporization and loss of the essential liquid components of the reaction admixture (e.g. excluding by-product alcohol).

Thus, while any effective temperature may be employed, it is contemplated that such effective temperatures typically will be at least 5°C, preferably at least 10°C, and most preferably at least 15°C, and can vary typically from about 5 to about 200°C, preferably from about 10 to about 150°C, and most preferably from about 15 to about 100°C.

The formula III compound precursor forming reaction time is selected in conjunction with the reaction temperature and the amounts of Hydrocarboxides I and II to permit substantially complete reaction at the above reaction temperatures. Such reaction times typically will vary from about 0.15 to about 40 hours, preferably from about 0.2 to about 30 hours, and most preferably from about 0.5 to about 20 hours, as measured from the initiation of contact of all of the reactive components of the admixture. It is desired to conduct admixture of Hydrocarboxides I and/or II with the acidic phosphorus oxygen compound to permit a slow reaction therebetween. This is typically achieved by controlling the addition times thereof to be between about 0.5 and about 15 hours. The reaction generally will be substantially complete after typically from about 0.3 to about 10, preferably from about 0.5 to about 8, and most preferably from about 0.5 to about 5 hours, measured from completion of the formation of the reaction admixture at ambient temperature. Higher reaction temperatures will cause completion of the reaction in shorter times.

The reaction pressure is not critical provided undue loss of the liquid contents of the reaction admixture is avoided, and can be atmospheric, subatmospheric or superatmospheric.

While not critical, upon passage of the aforedescribed reaction times and apparent completion of the reaction, it is preferred to allow the contents of the admixture to age for periods of typically from about 1 to about 30 hours, and preferably from about 2 to about 22 hours, e.g., at reaction temperatures of typically from about 10 to about 100°C to assure that complete reaction has occurred.

Upon completion of the reaction and optional aging the formula III catalyst precursor is separated from the organic medium. Generally, the organic medium is selected so that the catalyst precursor is insoluble

therein at room temperature. Thus, precursor separation can be accomplished in a variety of ways. Typically, it takes place in two stages, namely, by bulk separation and then final purification, e.g., by drying.

Bulk separation can be accomplished by filtering the reaction admixture to recover the catalyst precursor as a filter cake, by centrifuging the reaction admixture, and separating, e.g., by decanting, the supernatant liquid organic medium from the solid precursor, or by evaporating the liquid organic medium to form a cake or paste of the catalyst precursor.

The precursor solids, after bulk separation, are then typically subjected to conditions sufficient to remove any residual liquid organic medium or any organic contaminants. This can be achieved by drying, preferably continuous drying, to evaporate residual organic liquid medium, by washing the precursor solid with water or with an organic medium, preferably an organic medium, having a higher vapor pressure than the organic medium employed to form the admixture to facilitate drying, or by employing both procedures. Thus, before final purification is conducted, the separated catalyst precursor solids can be washed in a liquid organic medium one or more times to remove any residual unreacted materials and/or any other organic soluble species followed by a repetition of bulk separation procedures and then drying, although this is not required.

Drying can be achieved by heating the precursor, e.g. by exposing the precursor to air at a temperature of from about 20 to about 160°C for a period of from about 0.5 to about 30 hours or by placing it in a forced circulation oven maintained at a temperature typically between about 40 and about 250°C for about 0.5 to about 30 hours. Alternatively, the precursor can be air dried at room temperature for between about 1 to about 40 hours and then placed in the forced circulation oven until constant weight is attained. Drying under reduced pressure at room or elevated temperature, such as by using a vacuum oven is preferred.

The isolated formula III catalyst precursor composition is then calcined to form the final composition capable of catalyzing the formation of alpha, beta-unsaturated products described herein. Calcination can be conducted in a separate step or in-situ in the reactor and involves heating the precursor composition to a selected temperature or temperatures within a defined range. Preferably the calcination procedure is conducted in stages by heating the precursor in a stepwise fashion at increasingly higher temperature plateaus until a temperature of at least about 700°C is attained.

Accordingly, and in view of the above, calcination is conducted at temperatures of typically from about 600 to about 1300°C, preferably from about 650 to about 1000°C (e.g. 650 to 850°C), and most preferably from about 700 to about 1000°C (e.g. 700 to 950°C) for a period of typically from about 1 to about 48 hours, preferably from about 2 to about 30 hours, and most preferably from about 2.5 to about 20 hours. Most preferably, the final temperature plateau during calcination will be at least 720 to about 950°C for a period of about 0.5 to about 30 (e.g. 2 to 20) hours.

However, it is preferred to subject the precursor to a precalcination procedure by heating it at temperatures of typically from about 400 to about 599 and most preferably from about 450 to about 599°C, for periods of typically from about 0.1 to about 10, and preferably from about 0.5 to about 8 hours. Calcination and precalcination can be conducted as two different steps as by heating first at a selected precalcination temperature and then at a selected calcination temperature or by gradually increasing the temperature from a precalcination range to a calcination range.

The atmosphere under which the formula III catalyst precursor calcination is conducted includes oxygen or an oxygen containing gas such as air, nitrogen, helium, or other inert gas. At the higher calcination temperatures it is preferred to include oxygen in the calcination atmosphere.

While not essential, it is preferred that the calcination atmosphere be passed as a moving gaseous stream over the precursor composition.

Calcination can be conducted before, after, or during intermediate stages of shaping of the catalyst precursor.

While the above description of the method of preparing the preferred catalysts of formula III for use in the present invention is provided with respect to the minimum components which must be employed therein, it is contemplated that such catalysts may have other additives, (e.g. which modify the catalyst properties) or promoters incorporated therein which typically enhance the rate and/or selectivity of the intended reaction for which the catalyst will eventually be employed to catalyze. A preferred promoter for this purpose is boron. The preferred catalysts prepared as described hereinabove which contain boron exhibit slightly better activity at lower reaction temperatures when employed to catalyze the synthesis of the alpha, beta-ethylenically unsaturated products described herein. Boron can be incorporated into the catalyst composition during preparation of the catalyst precursor by impregnation of the catalyst precursor or catalyst with a suitable boron compound prior or subsequent to calcination procedures. Preferably, the boron compound is incorporated during preparation of the catalyst precursor. This can be achieved by selecting a suitable boron compound which preferably is soluble in the liquid organic medium. Representative examples of such boron compounds include boron acetate, boron hydrocarboxides, preferably boron alkoxides, wherein the hydrocarboxide portion is as described in connection with hydrocarboxides I and II, bis (di-acetoboron) oxide, boric acid, and mixtures thereof. The boron compound can be added to the precursor forming admixture directly or to any of the solutions which are combined to form the precursor forming admixture.

Alternatively, a boron compound can be impregnated into the catalyst composition by conventional means, such as by contact of the catalyst composition with an impregnating solution having the boron

compound dissolved therein. Compounds of titanium, such as, $TiO_2$ or titanium hydrocarboxide similar to the hydrocarboxides disclosed herein, can also be included in the catalyst in a similar manner.

The most preferred catalysts of formula III have a surface area of typically from about 40 to about 300, and preferably from about 50 to about 170 $m^2/g$, as determined by the BET method, the general procedures and theory for which are disclosed in H. Burnaur, P. Emmett and E. Teiler, J. of Am. Chem. Soc. Vol. 60, p. 309 (1938).

The catalysts for use in the process of the present invention (e.g. basic and/or acidic) are adaptable to use in the various physical forms in which catalysts are commonly used as particulate or powdered material in a contact bed, as a coating material on monolithic structures generally being used in a form to provide high surface area, as spheres, as extrudates, pellets and like configurations. The catalyst, can if desired, be composited with various catalyst binder or support materials, or physical property modifiers such as attrition resistance modifiers, which do not adversely affect the catalyst or the reactions in which the catalyst is to be employed.

Thus, various sized powders can be produced by grinding the catalyst to the desired size by any conventional or convenient means. Extrudates and pellets of various sizes and shapes can be prepared by using any conventional or convenient means. Utilizing a conventional screw type extruder, the dough or paste is processed through a die plate generally comprising orifice openings in the 1/32—1/2 inch diameter range to form generally cylindrical particles. The freshly extruded material may be collected in the form of strands of indefinite or random lengths to be dried and subsequently broken into extrudate particles; or the freshly extruded material may be cut into random or predetermined lengths of from about 1/4 inch to about 1/2 inch and subsequently dried; or the freshly extruded material may be formed into spheres, for example, by the process whereby the extrudate strands are collected in a spinning drum, the strands becoming segmented and spheroidized under the spinning influence of the drum.

In accordance with the process of the present invention, the reactants are contacted with an appropriate dehydration catalyst, preferably as a vaporous mixture. For ease of discussion, the reactants are designated as follows: the saturated carboxylic acid (i-a), the corresponding ester of the saturated carboxylic acid (i-e), and formaldehyde or derivative (ii). Thus, starting materials (i) and (ii) are employed in stoichiometric amounts or in excess of either one over the other. Accordingly, the mole ratio of starting materials (i) and (ii) typically can vary from about 1:2 to about 100:1, preferably from about 1:1 to about 50:1, and most preferably from about 5:1 to about 30:1.

The mole ratio of reactants (i-a) and (i-e) in the initial feed stream prior to contact and modification with ether can be any amount effective to bring about the subject condensation reaction. Thus, while any effective amount of said reactants can be employed, it is contemplated that such effective amount constitute a mole ratio of reactants i-e:i-a of typically from about 1:10 to about 500:1, preferably from about 1:5 to about 300:1, and most preferably from about 1:1 to about 200:1.

The product resulting from the aforedescribed condensation reaction will typically comprise a mixture of alpha, beta-ethylenically unsaturated acid (designated product i-a-P) and the corresponding alpha, beta-ethylenically unsaturated ester (designated i-e-P) as well as ether by-products derived from sources described hereinabove and unreacted reactants.

Upon commencement of the condensation reaction unreacted saturated acid reactant (i-a) and optionally alpha, beta-ethylenically unsaturated acid product (i-a-P) is preferably recovered from the effluent stream and recycled to the condensation reactor and/or esterified in a separate reactor with the ether. Thus, the feed stream to the condensation reactor can also comprise unsaturated acid product i-a-P.

When the ether is introduced into the feed stream of the condensation reactor, the amount of ether so introduced is a function in part of the amount of acid reactant (i-a) and acid product (i-a-P) present in such. Likewise, when the ether is introduced into a process stream intended as a feed to an esterification reactor as described hereinafter. Since the primary function of the ether is to increase the proportion of ester reactant (i-e) and ester product (i-e-P) in a given process stream relative to the respective corresponding free acids thereof in a given process stream, i.e. increase the mole ratios of i-e:i-a and i-e-P:i-a-P, the amount of ether introduced into a particular process stream can be expressed as a mole ratio of ether to the sum of the moles of reactant acid (i-a) and acid product (i-a-P) present in said process stream.

Thus, while any amount of ether effective to increase the mole ratios of reactants i-e:i-a and/or products i-e-P:i-a-P in a process stream can be employed, it is contemplated that such effective amounts be sufficient to constitute a mole ratio of ether: (i-a+i-a-P) in a proces stream of typically from about 1000:1 to about 1:200, preferably from about 500:1 to about 1:150, and most preferably from about 300:1 to about 1:100.

Additional ether can be employed as a water scavenging agent where desired. Likewise, water can be employed to control the amount of ether by hydrolysis of the ether to alcohol. In this way only the amount of ether needed to recycle can be employed while converting unused ether to a valuable alcohol product process credit as described hereinafter.

The amount of ether by-product available in-situ for recycle to a process stream will depend on the type of dehydration catalyst employed as well as the type of reactant (ii) employed. More specifically, acidic dehydration catalysts typically result in the production of more ether by-products than basic catalysts such as LiOH impregnated on $AlPO_4$, and $Li_3PO_4$ on silica gel, although basic catalysts also will produce ether by-product. Thus, the present invention finds particular application in the use of acidic condensation

catalysts. Furthermore, the use of methylal as reactant (ii) will also contribute to ether by-product formation. If necessary, additional ether can be generated from organic alcohols typically present in the process streams, such as methanol. The process of the present invention can be conducted continuously or batchwise, in a fixed or fluid bed. The catalyst may be charged to a tube or on trays or in a fluid bed, etc. through which the reactant mixture is passed. The reactor system may consist of a series of catalyst beds with optional interstage heating or cooling between the beds if desired. It is also an embodiment of the invention to use either upflow or downflow of the reactants through the reactor, with periodic reversal of gas flow also being contemplated to maintain a clean catalyst bed. If desired the gaseous feed may be charged together with an inert carrier gas, e.g., nitrogen, helium, argon, carbon oxides or low molecular weight hydrocarbons.

Furthermore, it has been found that when employing the preferred aluminum/silicon alkoxide derived catalysts described hereinabove, it is advantageous to introduce an aromatic hydrocarbon, such as toluene, xylene, benzene, and mixtures thereof into the feedstream to delay deposit of coke on the catalyst. Such aromatic hydrocarbons are typically employed in amounts of from about 1 to about 45 mole percent of the total feed to the condensation reactor.

The reaction temperature at which the subject condensation reactions is conducted will typically vary from about 230 to about 500°C, and preferably from about 270 to about 450°C, under atmospheric or superatmospheric pressures (e.g. 1 to 150 psig; 6.9 to 1034 kPag) although subatmospheric can also be employed.

Suitable feed rates of reactants to the condensation reaction zone typically can vary from about 0.1 to about 15, preferably from about 0.3 to about 10 and most preferably from about 0.5 to about 10 $hr^{-1}$ LHSV.

Representative process schemes are discussed below to illustrate various embodiments of the present invention using methyl propionate and/or propionic acid as the reactants and dimethyl ether as the recyclable by-product. It will be understood, that when methylal is employed as a reactant, a portion of the same typically will be converted to formaldehyde by-product which can be recovered and recycled if desired.

Figure 1 illustrates a process flow scheme wherein propionic acid and formaldehyde are employed as initial reactants. This flow scheme employs a methanol recycle in addition to a DME recycle. It will be understood that since propionic acid is less expensive than methyl propionate, there is a cost incentive to employ propionic acid in the initial feed stream which is then converted to methyl propionate in situ.

Thus, in accordance with Figure 1 propionic acid, formaldehyde and water, and methanol are introduced to line 4 via lines 1, 2 and 3, respectively. The resulting mixture in line 4 is passed to condensation reactor 8 via heat exchanger 5, lines 6 and 7. The condensation reactor contains an appropriate dehydration catalyst. Prior to entering the condensation reactor, the reactant mixture in line 6 is combined with recycle stream in line 36 containing methyl propionate, propionic acid, dimethyl ether and methanol. Reactor effluent exits the condensation reactor 8 via line 9, passes through heat exchanger 10 and is introduced into distillation column 12 via line 11. In distillation column 12, dimethyl ether is vaporized and passes from the top thereof via line 35. This process stream is then combined with the contents of line 31 and passed through heat exchanger 41 via line 32 to line 36. The bottoms of distillation column 12 are passed via line 13, heat exchanger 14 and line 15 to distillation column 16. In distillation column 16, methyl propionate, formaldehyde, methanol, and trace amounts of water are vaporized and passed out of the top of column 16 via line 34. The contents of this process stream are then combined with the contents of line 30 (containing PA) and the resulting mixture passed via line 31 for mixture with the contents of line 35 as described hereinabove. The bottoms from distillation column 16 are removed thereform via line 17, passed through heat exchanger 18 and into distillation column 20 via line 19. In column 20, MMA and water are vaporized and passed from the top of column 20 via line 33 to separator 37. MMA exits the separator via line 38, and water via line 39. The bottoms from column 20 are extracted therefrom via line 21, passed through heat exchanger 22 and into the distillation column 24 via line 23. In column 24, PA is vaporized, passed from the top thereof via line 30 and combined with the process stream 34 in line 31. The bottoms from column 24 are removed therefrom via line 25, passed through heat exchanger 26 and into distillation column 28 via line 27. In column 28, MA is vaporized and passed over the top thereof via line 40. The MA can then be recovered or passed for recycle via line 41 to line 4, for conversion to MMA. If necessary the amount of DME which is recycled to the condensation reactor can be controlled by vent line 42. Thus, in accordance with the process scheme of Figure 1, fresh PA and recycled PA is converted by reaction with DME in the condensation reactor to MP, and optionally, recycled MA is converted to MMA. The process scheme of Figure 1 is less preferred because the use of formaldehyde engenders large amounts of water associated therewith. Consequently, a large excess of DME is employed for its water scavenging properties.

The process scheme of Figure 2 employs PA and methylal as initial feed reactants and does not use a methanol recycle. More specifically, PA and methylal are introduced into the process scheme via lines 201 and 202 respectively. These materials are combined in line 203 and passed through the condensation reactor via line 204, heat exchanger 205 and line 206. Prior to introduction into the condensation reactor, PA and methylal in line 203 are combined with recycled methylal, MP and PA present in line 242, and recycled DME present in line 245. Reactor effluent exits condensation reactor 207 via line 208, is passed through heat exchanger 209 and enters distillation column 211 via line 210. In distillation column 211, DME is vaporized

and exits the top thereof via line 243 where it is either passed via line 245 for recycle back to condensation reactor 207 or vented via line 244. The bottoms in distillation column 211 exit the same via line 212, are passed through heat exchanger 213 and enter distillation column 216 via line 215. In column 216 unreacted methylal, if any, and methanol are vaporized and exit the top thereof via line 240. The contents of line 240 enter separator 239 wherein a small amount of methanol is removed from the bottom thereof via line 241 and methylal exits the top of separator 239 via line 238 where it is combined with the contents of line 237 and recycled to the condensation reactor via line 242. The bottoms of distillation column 216 are removed therefrom via line 217, passed through heat exchanger 218 and into distillation column 220 via line 219. In column 220, unreacted MP, and formaldehyde are vaporized and exit the top thereof via line 236. The contents of line 236 are combined with PA from line 234 and passed to line 237 for combination with the contents of line 238 and recycled to the condensation reactor. The bottoms from column 220 are extracted therefrom via line 221, passed through heat exchanger 222, and enter distillation column 224 via line 223. In column 224, MMA and water are vaporized and exit the top thereof via line 235. The contents of line 235 are passed to separator 246 where water and MMA are separated and exit the same via lines 248 and 247, respectively. The bottoms in column 224 exit the same via line 225, pass through heat exchanger 226 and into distillation column 228 via line 227. In column 228, PA is vaporized and exits the top of the same via line 234 where it is combined with the contents of line 236 for recycle back to the condensation reactor. The bottoms in column 228 are removed therefrom via line 229, passed through heat exchanger 230 and into distillation column 232 via line 231. In distillation column 232, MA is vaporized and exits the top thereof via line 249. Optionally, MA may be recycled back to the condensation reactor via line 250 for conversion to MMA. Heavys are discarded from the bottom of column 232 via line 233.

Figure 3 represents the most preferred process scheme. This embodiment employs an esterification reactor to convert both reactant (e.g. PA) and product (e.g. MA) acids to esters with DME. Furthermore, the hydrolysis of DME to methanol is an acid catalyzed reaction. Consequently, by using an acidic dehydration catalyst in the esterification reactor, excess DME, over and above that need to esterify the acid feed can be converted to methanol by appropriate control of recycled water entering the esterification reactor. In this way, unused DME is converted to a valuable process credit, i.e., methanol, which in turn can be converted to formaldehyde or sold.

Referring to Figure 3, fresh PA is introduced into the process via line 340 where it is mixed with recycled PA from line 338 and recycled MA from line 336. The resulting mixture is passed to line 360 and further mixed with recycled DME from line 351. THe DME, PA and MA mixture is then passed via line 341 through heat exchanger 363 and introduced into the esterification reactor via line 359. Optionally, recycled water from line 343 can be mixed with the contents of line 342 to control the amount of excess DME in the esterification reactor through hydrolysis of the same. The contents of the esterification reactor exit the same via line 308 and are combined with the contents of line 307 in line 309. Fresh methylal enters the system via line 301, is mixed with recycled methylal from line 350 in line 302, and is passed via line 303, heat exchanger 304 and line 305 into condensation reactor 306. Prior to entering condensation reactor 306, the contents of line 302 are mixed with recycled MP from line 346, and recycled DME from line 355. The contents of condensation reactor 306 exit the same via line 307, are combined with the contents of line 308 in line 309, passed through heat exchanger 310 and enter distillation column 312 via line 311. In distillation column 312, DME is vaporized and exits the same via line 354. A portion of the DME in line 354 is passed via line 355 to line 303 for recycle to the condensation reactor 306, and the remainder is passed via line 352 and line 351 to line 341, wehre it is mixed with the contents of line 360 as described above for recycle to the esterification reactor. Excess DME can be vented via line 353. The bottoms in column 312 exit the same via line 313, are passed through heat exchanger 314 and enter distillation column 316 via line 315. In column 316, unconverted methylal, if any, and methanol are vaporized and exit the same via line 348 and are then introduced to separator 347. In separator 347, methylal is vaporized and exits the same via line 350 where it is recycled to line 302 for introduction into the condensation reactor. Methanol is discharged via line 349 from the bottom of separator 347. The bottoms from distillation column 316 exit the same via line 317, are passed through heat exchanger 318 and into distillation column 320 via line 319. In column 320, unreacted MP, and formaldehyde are vaporized and exit the same via line 346. The contents of line 346 are mixed with the contents of lines 302 and 355 for recycle into the condensation reactor. The bottoms of column 320 exit the same via line 321, are passed through heat exchanger 322 and into distillation column 324 via line 323. In column 324, MMA and water are vaporized and exit the same via line 339. The contents of line 339 are introduced into separator 361. MMA exits separator 361 via line 356, and water exits the separator via line 357. Optionally, a portion of the water in line 357 can be passed via line 358, through heat exchanger 344, and into line 343 for recycle back to esterification reactor 362 via line 359. The bottoms in column 324 exit the same via line 325, are passed through heat exchanger 326, and into distillation column 328 via line 327. In column 328, PA is vaporized, and exits the same via line 337, and is mixed with the contents of line 336 in line 338. The bottoms of column 328, exit the same via line 329, are passed through heat exchanger 330, and enter distillation column 332 via line 331. In column 332, MA is vaporized and exits the same via line 334. All or a portion of the MA in line 334 is then passed to line 336 and mixed with the PA of line 337 in line 338 for recycle to the esterification reactor, in combination with the DME in line 351, via line 341. Excess MA is discharged from the system via line 335. Heavys are discharged from column 332 via line 333 and discarded. It is to be understood that the acid catalyst employed in condensation reactor 306 may be either

acidic or basic, preferably acidic, while the catalyst employed in esterification reactor 362 must be acidic to permit the hydrolysis of excess DME with recycled water in addition to the esterification reaction.

Figure 4 illustrates a process scheme similar to that depicted in Figure 3 with the exception that methyl propionate and methylal are employed as the initial reactants. However, unreacted PA and MA are esterified in a separate esterification reactor rather than recycled back to the condensation reactor as depicted in Figures 1 and 2. Thus, the advantages and benefits of the esterification reactor discussed in connection with Figure 3 are also achieved in the process scheme of Figure 4. More specifically, MP and methylal are introduced into the process via lines 401 and 402 respectively, passed to line 403 where they are mixed, and then passed to the condensation reactor 407 via lines 404, heat exchanger 405 and line 406. Prior to introduction into the condensation reactor, however, the contents of line 403 are mixed with the contents of line 450 which contains recycled DME, methylal, formaldehyde and MP. The introduction of DME to condensation reactor 407 serves to esterify any acid resulting from hydrolysis of ester reactants and/or products.

The contents of condensation reactor 407 exit the same via line 408 are mixed with the contents of line 409 in line 410, passed through heat exchanger 411, and into distillation column 413 via line 412. In distillation column 413, DME is vaporized and exits the same via line 452 where a portion of it is recycled to condensation reactor 407 via line 464, the contents of which are mixed with the contents of line 449 in line 450. The remainder of DME in lien 452 is passed via line 453 and line 441 to line 442. The DME in line 441 is mixed with PA and MA from line 439 in line 442. The resulting mixture is passed through heat exchanger 443, line 444 and 446 and into esterification reactor 447. Vent line 454 permits discharge of excess DME. The bottoms of distillation column 413 are discharged from the same via line 414, passed through heat exchanger 415 and into distillation column 417 via line 416. In distillation column 417, methylal and methanol are vaporized and exit the same via line 463 and pass into separator 461. Methylal is discharged from the top of separator 461 via line 451 and mixed with the contents of line 448 (MP) in line 449 for recycle to the condensation reactor. Methanol is discharged from the bottom of separator 461 via line 462. The bottoms of distillation column 417 exit the same via line 418, are passed through heat exchanger 419 and into distillation column 421 via line 420. In column 421, MP and formaldehyde are vaporized, discharged from the same via line 448 and mixed with the methylal from line 451 in line 449 for recycle to the condensation reactor. The bottoms of column 421 are discharged from the same via line 422, passed through heat exchanger 423 and into distillation column 425 via line 424. In column 425, MMA and water are vaporized and discharged from the same via line 440, and passed into the separator 455. MMA and water are discharged from the separator via lines 456 and 457 respectively. A portion of the water from line 457 may be passed via line 458 through heat exchanger 460 and line 445 into esterification reactor 447 after combination with the contents of line 444 in line 446. Thus, water is employed to control the amount of DME in the esterification reactor through hydrolysis of the DME. The bottoms in column 425 exit the same via line 426, are passed through heat exchanger 427, and into distillation column 429 via line 428. In column 429, PA is vaporized and exits the same via line 438. The PA in line 438 is optionally combined with MA from line 437 in line 439. The contents of line 439 are then combined with the DME from line 441 for recycle to the esterification reactor. The bottoms of column 429 exit the same via line 430, are passed through heat exchanger 431, and into distillation column 433 via line 432. In column 433, MA is vaporized and exits the same via line 435. A portion of the MA in line 435 may be passed through line 437 to line 439 to the esterification reactor for recycle and/or discharged via line 436. Heavys from column 433 are discharged from the same via line 434. The contents of esterification reactor 447 are discharged from the same via line 409. If MA has been recycled via line 437 to the esterification reactor, the contents of line 409 typically will contain MP, MMA, methanol, DME, water, unreacted MA and unreacted PA. The contents of line 409 are then mixed with the discharge from the condensation reactor present in line 408, in line 410.

The schematic showing of Figures 1 to 4 omit many features which those skilled in the art would recognize as desirable or essential in actual plant operation. These omissions are made in order to simplify the presentation of the invention and to avoid encumbering it with well understood engineering details. Furthermore for ease of review, while Figures 1 to 4 identify the primary components of a particular process stream where space permits, the recitation of such components in a figure is not intended to be exhaustive, with reference to the specification being required to supplement such recitation.

The following examples are given as specific illustrations of the claimed invention. It should be understood, however, that the invention is not limited to the specific details set forth in the examples. All parts and percentages in the examples as well as in the remainder of the specification are by weight unless otherwise specified.

In the following examples, unless otherwise specified, each catalyst is tested in the following manner: A glass tube reactor 20 inches (50.8 cm) in length, 1 inch (2.54 cm) O.D. and about 0.8 inch (2.03 cm) I.D., is stoppered at the bottom with glass wool, loaded with 20 cc of catalyst sample, on top of which is placed 10 cc of glass wool, followed by the addition of a sufficient number of 4 mm diameter glass balls to fill the remaining reactor tube volume. The glass balls serve as a preheating zone about 7 inches in length within the tube. The reactor is then mounted in a vertical furnace having a heating chamber 2.5 cm in diameter and 30.5 cm in length. A liquid reactant feed stream is then passed downward through the reactor tube at a selected furnace temperature as described herein. The feed stream is vaporized in the preheating zone and contacts the catalyst as a vapor. All reactions are carried out under ambient atmospheric pressure. The

reactant feed stream is passed through the reactor at a liquid hourly space velocity (LHSV), as described herein. The reactor effluent for the first 15 minutes after each start-up is discarded, but is collected thereafter for a period of 2.5 hours in an ice trap. The total liquid effluent collected during this time is analyzed by gas chromatography, mass spectrophotometry, and NMR. Analysis for formaldehyde, other aldehydes, and ketones, if made, is conducted by reacting the respective reaction products with o-benzylhydroxylamine hydrochloride and sodium acetate, said reaction being conducted in the presence of at least 55%, by weight methanol based on the weight of the mixture.

Unless otherwise specified, conversion of methylal, selectivity, and yield are calculated as follows:

$$\text{Methylal conversion } (\%) = \frac{A-B}{A} \times 100$$

$$\text{Selectivity to MMA+MA}(\%) = \frac{C}{A-B-D} \times 100$$

$$\text{Yield of MMA+MA } (\%) = \frac{C}{A} \times 100$$

wherein the above equations:

A=moles of methylal in feed.
B=moles of methylal in reaction product.
C=moles of MMA+MA in reaction product.
D=moles of formaldehyde in reaction product.
MMA=methyl methacrylate.
MA=methacrylic acid.

Furthermore as used herein reaction efficiency is defined as follows:

$$\text{Reaction efficiency } (\%) = \frac{\text{Moles of MMA and/or MA in product}}{\text{Moles of MMA and/or MA in feed}} \times 100$$

Thus, the reaction efficiency of a particular material expresses the change in moles of that material after contact with the catalyst as a percentage of the moles of that material in the feed.

Example 1

The following example illustrates the preparation of an acidic Si/Al/P/O dehydration catalyst to be used in other examples.

Two solutions were prepared. In the first solution 155.15 g of tetraethyl orthosilicate ($Si(OC_2H_4)_4$) and 115 g of aluminum tri-sec-butoxide ($Al(OC_4H_9)_3$) were dissolved in 1100 cc of acetone. The second solution was prepared by dissolving 50.25 g of an 85% aqueous solution of $H_3PO_4$ and 45.61 g of water in 250 cc of acetone. The second solution was slowly added at 25°C to the first solution over a period of 5 hours with continuous vigorous mechanical stirring. A white precipitate was separated from the reaction mixture by filtration and the precipitate dried in air at 115°C overnight in a vacuum oven. The dried solid was then calcined in air at 430°C for 2 hours, and 520°C for 4 hours. The calcined product was ground to a powder −20+48 mesh (Tyler sieve series). The powder was mixed and pelletized to 1/8×1/8 inch size pellets. The pellets were then calcined in air at 600°C for 18 hours, 750°C for 5.5 hours, 820°C for 6 hours, and then 880°C for 5 hours. The resulting catalyst sample is designated Sample A.

Example 2

The following example illustrates the preparation of an acidic Si/Al/Ti/P/O catalyst for use hereinafter.

Two solutions were prepared in general accordance with Example 1 using 141.99 g of tetraethyl orthosilicate, 42.41 g of titanium tetrabutoxide, 142.41 g of aluminum tri-sec-butoxide, and 1150 cc of acetone for solution 1, and 62.23 g of 85% aqueous $H_3PO_4$ solution, 48.20 g of water and 300 cc of acetone for solution 2. The two solutions were combined in accordance with Example 1 to form a reaction mixture. The reaction mixture was then aged for 18 hours at 25°C with mechanical stirring. A white precipitate was separated from the reaction mixture by filtration, the filter cake dried at 100°C overnight in a vacuum oven, and then calcined at 400°C for 1.5 hours, 500°C for 1 hour, and 560°C for 3 hours in air. The calcined product was ground to a powder (−16 mesh) and 40.5 g thereof mixed with 7 g of water and then pelletized. The pellets (0.5 inch diameter) were calcined at 120°C for 1.5 hours, 300°C for 1 hour, and 750°C for 3 hours in air. The calcined product was ground to −6+16 mesh granules. The resulting catalyst sample is designated Sample B.

Example 3

The following example illustrates the preparation of an acidic Si/Al/P/O catalyst used hereinafter.

In general accordance with Example 1, two solutions were prepared using 137.71 tetraethyl orthosilicate, 57.21 g aluminum tri-sec-butoxide, and a mixture of 1050 g of diethyl ether and 500 cc of acetone for solution 1, and 26.34 g of the 85% aqueous $H_3PO_4$ solution, 25.54 water and 200 cc of acetone for solution 2. In addition, a third solution was prepared by dissolving 25.43 of aluminum tri-sec-butoxide in 150 cc of acetone; and a fourth solution was prepared by dissolving 11.56 of the 85% aqueous $H_3PO_4$ solution and 12.64 g of water in 100 cc of acetone. Solution 2 was then gradually added to solution 1 over a period of about 4 hrs at room temperature while stirring vigorously, to form a reaction mixture. Solution 3 was then slowly added to the continuously agitated reaction mixture over a period of 5 min; and the reaction mixture stirred for an additional 0.97 hrs upon completion of the solution 3 addition. Solution 4 was then slowly added to the continuously agitated reaction mixture over a period of 1 hr and the contents of the reaction mixture refluxed for 1.13 hours from completion of the solution 4 addition. The reaction mixture was then cooled to room temperature and aged for 18 hours at room temperature under continuous mechanical agitation. A white precipitate was separated from the reaction mixture by filtration, and the filter cake dried at 110°C for 18 hours in a vacuum oven. The dried filter cake was then calcined at 450°C for 1 hour, and 528°C for 4 hours in air. The calcined filter cake was ground to a powder (−16 mesh) and 28.92 g thereof, mixed with 1.0 g of water soluble starch, and then 8.3 g water. The mixture was pelletized and the pellets (0.5 inch diameter) calcined at 450°C for 1 hour, and 600°C for 3.3 hours in air. The calcined pellets were ground to −6+16 mesh granules, and the granules further calcined in air at 600°C for 1 hour, 750°C for 4 hours, 820°C for 4.5 hours, and 880°C for 4 hours. The resulting catalyst sample is designated Sample C.

Example 4

This example illustrates the effect of contacting MMA, MA and MP with a dehydration catalyst in the absence of water under reaction conditions.

Thus, 10 cc (3.61 g) of catalyst Sample A from Example 1 was loaded into the reactor as described hereinabove. A feed comprising 9.87 wt. % MMA, 12.35 wt. % MA, and 77.78 wt. % MP was passed through the reactor at a feed rate of 2.0 $hr^{-1}$ (LHSV) at two different temperatures as shown at Table 1, namely 300°C (Run 1) and 320°C (Run 2). Product effluent was analyzed as described above and the reaction efficiencies calculated as shown at Table 1 for Runs 1 and 2.

Example 5

Example 4, Run 2, was repeated using a feed comprising a solution of 8.72 wt. % MMA, 13.27 wt. % MA, dissolved in 78.01 wt. %, benzene based on the weight of these three components. The three component feed mixture was then saturated with water, to the point where only one phase was observed. The product effluent was analyzed and the results, in terms of reactive efficiencies, are reported at Table 1, Run 3.

Example 6

Five cc of catalyst Sample A were loaded into the reactor and a feed comprising a solution of 76.0 wt. % MP, 10.07 wt. % MMA, 11.68 wt. % MA, and 2.25 wt. % $H_2O$ was passed therethrough at a feed rate of 2.0 LHSV and a reaction temperature of 300°C (Run 4) and 320°C (Run 5). The reaction efficiencies are reported at Table 1.

Example 7

Twenty cc (10.40 g) of catalyst Sample B from Example 2 was placed in the reactor and a feed comprising 10 wt. % methylal, and 90 wt. % MP, was passed therethrough at a feed rate of 1.0 LHSV and 350°C reaction temperature. The product effluent was analyzed for conversion and yield of MMA+MA and the results summarized at Table 2, Run 6.

Upon completion of Run 6, the catalyst Sample B was re-calcined in air at 600°C for 1 hour, and then 820°C for 4—5 hours. A 20 cc (6.96 g) sample of the re-calcined catalyst was again loaded into the reactor and heated to 350°C while a feed comprising 74.41 wt. % MP, 9.11 wt. % PA, and 15.48 wt. % methanol was passed therethrough at a feed rate 1 LHSV. The reactor effluent was analyzed to a limited extent and the results summarized at Table 2, Run 7. The product analysis showed that no detectable decarboxylation of MP and/or PA occurred, i.e. no $CO_2$ was observed.

Upon completion of Run 7, the catalyst sample was heated at 350°C while passing $N_2$ gas through the reactor for 3.5 hours.

The following experiment was then carried out with this catalyst. The reactor was heated to 320°C and feed comprising 35.05 wt. % diethylether and 64.95 wt. % PA was passed therethrough at a feed rate of 1 LHSV. The product analysis showed 38.7 mole % of PA was converted to ethyl propionate without any indication of decarboxylation of PA and/or ethyl propionate. This experiment is designated Run 8 and the results summarized at Table 2.

Example 8

Twenty cc (6.34 g) of fresh catalyst Sample C were placed into the reactor for each of Runs 9 and 10. The following two runs were conducted using two different feed compositions which varied with respect to the amount of MP and PA.

The reaction conditions are summarized at Table 2 at Run 9 (feed: 10 wt.% methylal and 90 wt.% MP) and Run 10 (feed: 10 wt. % methylal and 90 wt. % PA). The results of the product analysis are also summarized at Table 2.

Example 9

Twenty cc of catalyst Sample A were loaded into the reactor for each of the following Runs 11 to 13. Each run employed a different feed composition as shown at Table 2, Runs 11 to 13. The reaction conditions and results of product analysis are also summarized at Table 2 for the respective runs.

TABLE 1

| Run No. | Corresponding Example No. | Catalyst sample | Testing conditions: Feed stream* | Testing conditions: Furnace temp. (°C) | Testing conditions: Feed rate (LHSV) | Reaction efficiency |
|---|---|---|---|---|---|---|
| 1 | 4 | A | A | 300 | 2 | 99.46 (MMA+MA) |
| 2 | 4 | A | A | 320 | 2 | 99.42 (MMA+MA) |
| 3 | 5 | A | B | 320 | 2 | 93.3 (MMA)<br>71.5 (MA)<br>79.2 (MMA+MA) |
| 4 | 6 | A | C | 300 | 2 | 99.1 (MMA+MA) |
| 5 | 6 | A | C | 320 | 2 | 99.0 (MMA+MA) |

* Feed compositions.
A=MMA (9.87 wt. %)+MA (12.35 wt. %)+MP (77.78 wt. %).
B=MMA (8.72 wt. %)+MA (13.27 wt. %)+benzene (78.01 wt. %) saturated with $H_2O$.
C=MP (76.0 wt. %)+MMA (10.1 wt. %)+MA (11.7 wt. %)+$H_2O$ (2.25 wt. %).
MA=methacrylic acid.
MMA=methyl methacrylate.

TABLE 2

| Run No. | Corres-ponding Ex. No. | Catalyst sample | Reaction conditions: Reactant* feed stream | Reaction conditions: Furnace temp. (°C) | Reaction conditions: Feed rate (LHSV) | Methylal conversion (%) | Yield of MMA+MA (%) | Selectivity to MMA+MA (%) | Mole ratio MMA:MA in product | Mole ratio MP:PA in product | Mole ratio MP:PA in feed |
|---|---|---|---|---|---|---|---|---|---|---|---|
| 6 | 7 | B | A | 350 | 1 | 100 | 51.0 | N/D | 7.8:1 | 8.5:1 | 1:0 |
| 7 | 7 | B | B | 350 | 1 | N/A | N/A | N/A | N/A | N/A | 7.2:1 |
| 8 | 7 | B | C | 320 | 1 | N/A | N/A | N/A | N/A | .6:1** | 0:1** |
| 9 | 8 | C | A | 350 | 1 | 100 | 69.1 | 97.8 | 6.7:1 | 6.19:1 | 1:0 |
| 10 | 8 | C | D | 350 | 1 | 100 | 62.2 | 81.1 | 1:6.0 | 0.24:1 | 0:1 |
| 11 | 9 | A | A | 320 | 1 | 100 | 75.7 | 84.3 | 6.7:1 | 5.7:1 | 1:0 |
| 12 | 9 | A | D | 320 | 1 | 100 | 44.8 | 58.0 | 0.11:1 | 0.23:1 | 0:1 |
| 13 | 9 | A | E | 320 | 1 | 100 | 60.4 | 78.4 | 5.2:1 | 4.75:1 | 1:0 |

* Feed compositions:
A=methylal (10 wt. %)+MP (90 wt. %).
B=MP (75.41 wt. %)+PA (9.11 wt. %)+methanol (15.48 wt. %).
C=diethyl ether (35.05 wt. %)+PA (64.95 wt. %).
D=methylal (10 wt. %)+PA (90 wt. %).
E=methylal (10 wt. %)+MP (53.5 wt. %)+benzene (36.5 wt. %).

** mole ratio of EP:PA
PA=propionic acid.
MP=methyl propionate.
MMA=methyl methacrylate.
MA=methacrylic acid.
DEE=diethyl ether.
EP=ethyl propionate.
N/A=not applicable.
N/D=not determined.

Discussion of results

Referring to Table 1, Runs 1 and 2 illustrate that in the absence of water, contact of the MMA and MA with the catalyst does little to affect these materials at reaction temperature, and the reaction efficiencies are nearly 100%. However, when MP in the feed of Run 2 is replaced with inert benzene (in Run 3) saturated with water the reaction efficiency of MMA and MA are both decreased. However, much more MA is destroyed than MMA is illustrated by reaction efficiencies of MA and MMA of 71.5% and 93.3% respectively. Thus approximately 30 mole % of the MA is decomposed while only about 7 mole % of the MMA is decomposed. Since PA in a reactant feed will form primarily MA, while MP in the reactant feed will form primarily MMA, the discovery of the above reaction efficiencies: (a) clearly renders the use of MP in the feed more desirable than PA, to enhance MMA production directly, and (b) renders the conversion of MA to MMA in-situ desirable due to the inherent enhanced stability of MMA relative to MA.

Runs 4 and 5 replace the inert benzene of Run 3 with MP which acts as a water binding agent in the absence of formaldehyde or methylal. Thus the extensive decomposition of MA illustrated in Run 3 is believed to be caused by even small amounts of water in the reaction system. Unfortunately, the control of water is difficult because it is a by-product of the condensation reaction as illustrated by reactions 1, 5, 7 and 8. While MP may be a successful water binding agent, it is converted to PA by exerting this effect. Consequently, if MP is the sole water scavenging agent in a reaction mixture, containing formaldehyde or methylal, one has to accept the production of more MA at the expense of MMA. The above disadvantages are obviated by employing an ether by-product to not only scavenge water but also to convert fresh and/or recycled PA to MP as well as any PA formed by hydrolysis of MP back to MP.

Referring to Table 2, Run 6 illustrates particular advantage of the most preferred catalyst, namely, high activity at low reaction temperatures and no decarboxylation of MP at the employed reaction temperature. Run 7 illustrates that no decarboxylation occurs by the absence of $CO_2$ in the effluent (see Example 7 discussion).

Run 8 illustrates the ability of diethyl ether to convert significant amounts of PA to ethyl propionate in the presence of the same dehydration catalyst employed in Run 6. Note that diethylether (DEE) was employed rather than DME, in this Run, for convenience because DEE is a liquid at room temperature and more easily handled on a laboratory scale than DME. Thus, it is concluded from Runs 6 and 8 that if DME or DEE is recycled to the condensation reactor, the esterification of PA and MA will occur simultaneously with the condensation reaction to give better selectivity to the ester condensation products relative to the absence of DME or DEE.

Runs 9 and 10 contrast the effect of varying MP:PA mole ratios on the resulting condensation product. Thus, discounting for that portion of methylal which was converted to unreacted formaldehyde, the selectivity to MAA+MA using MP as the reactant was 97.8%. Furthermore, the mole ratio of MMA:MA was 6.7:1, indicating high selectivity to MMA. In contrast, the use of PA as reactant in Run 10 causes a substantial drop in MMA+MA selectivity to 81.1% and a decrease in the MMA:MA mole ratio to 1.0:6.0. Each of the selectivities of Runs 9 and 10 respectively are associated with high (6.19) and low (.24), MP:PA mole ratios in the product. In the present invention such ratios are maintained high with the use of ether by-product and similar results are shown for Runs 11 and 12.

Run 13 illustrates the effect of diluting some of the MP in Run 11 with inert benzene, namely, the selectivities and yields are intermediate to Runs 11 and 12. The replacement of some MP (a water scavenger through hydrolysis) with benzene in Run 13 increases the PA and MA concentrations in the reaction zone (due to increased MP and MMA hydrolysis) to levels intermediate to those employed in Runs 11 and 12. Consequently, one would expect selectivities intermediate to Runs 11 and 12 also. This expectation was therefore confirmed.

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

**Claims**

1. In a process for reacting a vaporous mixture comprising (a) at least one member selected from the group consisting of saturated carboxylic acid, and an ester of said saturated carboxylic acid and (b) at least one member selected from the group consisting of formaldehyde and a derivative of formaldehyde, in the presence of a dehydration catalyst, to form by a condensation reaction, a product comprising at least one member selected from the group consisting of alpha, beta-ethylenically unsaturated acid and an ester of said unsaturated acid, the improvement comprising introducing at least one ether into said vaporous mixture in a manner and under conditions sufficient to cause an increase in the proportional distribution of said alpha, beta-ethylenically unsaturated acid ester in said product relative to the absence of said ether introduction; said ether being characterized by the ability to: (a) convert said carboxylic acid reactant to its corresponding ester by reaction therewith; (b) convert said alpha, beta-ethylenically unsaturated acid product to its corresponding ester by reaction therewith; and optionally (c) hydrolyze under acidic reaction conditions in the presence of water to its corresponding alcohol.

2. The process of claim 1 wherein said dehydration catalyst is basic.

3. The process of claim 1 wherein said dehydration catalyst is acidic.

4. The process of claim 1 wherein said ether is formed as a by-product during said condensation reaction.

5. The process of claim 4 wherein said ether by-product is separated from said condensation reaction product and recycled for introduction into said vaporous mixture.

6. The process of claim 1 wherein said vaporous mixture comprises at least one compound represented by the structural formula:

$$R—CH_2—COOR'$$

wherein R and R′ are independently selected from the group consisting hydrogen, alkyl, aryl, aralkyl, and alkaryl.

7. The process of claim 6 wherein R is other than hydrogen.

8. The process of claim 6 wherein R and R′ are independently $C_1$ to $C_5$ alkyl.

9. The process of claim 6 wherein said ether is represented by the structural formula:

$$R'—O—R'$$

wherein R′ is as described in conjunction with the structural formula of claim 6 where R′ is other than hydrogen.

10. The process of claim 1 wherein said vaporous mixture comprises at least one compound selected from the group consisting of formaldehyde, methylal, and the hemiacetal of formaldehyde.

11. The process of claim 1 wherein said vaporous mixture comprises (a) at least one reactant selected from the group consisting of propionic acid and methylpropionate; and (b) at least one reactant selected from the group consisting of formaldehyde, methylal, and the hemiacetal of formaldehyde; and said ether which is introduced into said vaporous mixture is dimethyl ether.

12. The process of any of claims 1 to 11 wherein said dehydration catalyst is acidic and prepared by the process which comprises:

(1) reacting in admixture at least one Metal Hydrocarboxide I, at least one Metal Hydrocarboxide II, at least one acidic phosphorous-oxygen containing compound, and water, in the presence of at least one liquid organic medium comprising at least 50% by weight, based on the weight of said medium, of at least one member selected from the group consisting of organic aldehyde, organic ketone, and organic ether, said reaction being conducted in a manner sufficient to (a) avoid contact of Metal Hydrocarboxides I and II with water prior to contact of Metal Hydrocarboxide I and II with the acidic phosphorus-oxygen containing compound, and (b) form a catalyst precursor composition;

(2) separating said catalyst precursor composition from said reaction admixture;

(3) calcining said catalyst precursor composition to form said catalyst composition; wherein said process:

(i) the metal $M^1$, of said Metal Hydrocarboxide I is selected from at least one member of the group consisting of Al, Ga, In, and Tl; and

(ii) the metal, $M^2$, of said Metal Hydrocarboxide II is selected from at least one member of the group consisting of Si, Sn, Zr and Ge.

13. The process of claim 12 wherein in said catalyst preparative procedure, Metal Hydrocarboxide I is at least one aluminum alkoxide, said Metal Hydrocarboxide II is at least one silicon alkoxide, and the acidic phosphorus-oxygen compound is phosphoric acid.

14. The process of claim 12 wherein said vaporous mixture additionally comprises at least one aromatic compound to reduce coke formation on said catalyst.

15. A process for producing an alpha, beta-ethylenically unsaturated acid and ester thereof which comprises:

(a) providing an admixture comprising at least one saturated monocarboxylic acid, the methyl ester of said saturated carboxylic acid, aqueous formaldehyde and methanol;

(b) reacting said admixture, in the vapor phase and in at least one reaction zone, in the presence of a dehydration catalyst in a manner and under conditions sufficient to form an effluent mixture comprising water, the corresponding alpha, beta-ethylenically unsaturated acid and methyl ester derivative thereof, dimethylether, unreacted saturated monocarboxylic acid, and the unreacted methyl ester of said saturated monocarboxylic acid;

(c) separating and recovering dimethyl ether from said effluent mixture and recycling at least a portion of the recovered dimethyl ether to at least one of said reaction zones for combination with said admixture of step (a) in a manner and in an amount sufficient to convert at least a portion of the saturated monocarboxylic acid present in said admixture to its corresponding methyl ester; said effluent mixture after said recovery of dimethyl ether therefrom being designated residual effluent mixture;

(d) recovering at least a portion of the unreacted saturated monocarboxylic acid present in the residual effluent mixture and reacting at least a portion of said recovered saturated acid with at least a portion of

said dimethyl ether recovered in accordance with step (c) to form the corresponding methyl ester derivative of said acid and recovering said ester;

(e) recovering at least a portion of the unreacted methyl ester of the saturated monocarboxylic acid present in the residual effluent mixture;

(f) reacting at least a portion of the saturated monocarboxylic methyl ester, recovered in accordance with one or more of steps (d) and (e), with formaldehyde in the presence of a dehydration catalyst, methanol, and in the vapor phase to produce the corresponding alpha, beta-ethylenically unsaturated methyl ester thereof;

(g) recovering the alpha, beta-ethylenically unsaturated acid and methyl ester derivative thereof produced in accordance with steps (a) through (f).

16. The process of claim 15 wherein the reaction of said saturated acid with dimethylether in accordance with step (d) is conducted by recycling said saturated acid, and sufficient dimethyl ether recovered in accordance with step (c), for combination with said admixture of step (a), and reaction in said reaction zone of step (b).

17. The process of claim 15 wherein the reaction in accordance with step (f) is conducted by recycling at least a portion of the saturated ester recovered in accordance with one or more of steps (d) and (e) for combination with the admixture of step (a) and reaction in said reaction zone of step (b).

18. The process of claim 15 wherein at least a portion of the alpha, beta-ethylenically unsaturated acid recovered in accordance with step (g) is reacted with sufficient dimethyl ether to form the corresponding methyl ester of said unsaturated acid.

19. The process of claim 18 wherein said reaction is conducted by recycling said unsaturated acid and sufficient dimethyl ether recovered in accordance with step (c) for combination with the admixture of step (a) and reaction in the reaction zone of step (b).

20. The process of claim 15 wherein said saturated monocarboxylic acid is propionic acid.

21. The process of claim 15 wherein said dehydration catalyst is acidic.

22. The process of claim 15 wherein sufficient dimethyl ether is introduced into the reaction zone of step (b) to also cause hydrolysis of said ether to methanol thereby scavenging at least a portion of the water present in said reaction zone.

23. A process for producing alpha, beta-ethylenically unsaturated acid and ester thereof which comprises:

(a) providing an admixture comprising at least one saturated monocarboxylic acid, and methylal;

(b) contacting said admixture in the vapor phase in at least one reaction zone with an acidic dehydration catalyst in a manner and under conditions sufficient to react said admixture and produce an effluent mixture, comprising water, the alpha, beta-ethylenically unsaturated acid condensation product of methylal and said saturated acid, the corresponding methyl ester derivative of said unsaturated acid, dimethyl ether, unreacted saturated monocarboxylic acid, and optionally the methyl ester derivative thereof;

(c) separating and recovering dimethyl ether from said effluent mixture and recycling at least a portion of said ether to at least one of said reaction zones for combination with said admixture of step (a) in an amount sufficient to convert at least a portion of said saturated monocarboxylic acid present in said admixture to its corresponding methyl ester; said effluent mixture after recovery of dimethyl ether therefrom being designated residual effluent mixture;

(d) recovering at least a portion of the unreacted saturated monocarboxylic acid present in the residual effluent mixture and reacting at least a portion of said recovered saturated acid with at least a portion of the dimethyl ether recovered in accordance with step (c), to form the corresponding methyl ester derivative of said acid, and recovering said ester;

(e) optionally, recovering at least a portion of the methyl ester of the saturated monocarboxylic acid present in the residual effluent mixture;

(f) reacting at least a portion of the saturated monocarboxylic methyl ester recovered in accordance with one or more of steps (d) and (e) with methylal in the presence of a dehydration catalyst in the vapor phase to produce the corresponding alpha, beta-ethylenically unsaturated acid ester condensation product thereof;

(g) recovering the alpha, beta-ethylenically unsaturated acid and methyl ester derivative thereof produced in accordance with steps (a) through (f).

24. The process of claim 23 wherein the reaction of said saturated acid with dimethyl ether in accordance with step (d) is conducted by recycling said saturated acid, and sufficient dimethyl ether recovered in accordance with step (c), for combination with said admixture of step (a), and reaction in said reaction zone of step (b).

25. The process of claim 23 wherein the reaction in accordance with step (f) is conducted by recycling the saturated ester recovered in accordance with one or more of steps (d) and (e) for combination with the admixture of step (a) and reaction in said reaction zone of step (b).

26. The process of claim 23 wherein at least a portion of the alpha, beta-ethylenically unsaturated acid recovered in accordance with step (g) is reacted with sufficient dimethyl ether to form the corresponding methyl ester of said unsaturated acid.

27. The process of claim 26 wherein said reaction is conducted by recycling said unsaturated acid and

sufficient dimethyl ether recovered in accordance with step (c) for combination with the admixture of step (a) and reaction in the reaction zone of step (b).

28. The process of claim 23 wherein said saturated monocarboxylic acid is propionic acid.

29. The process of claim 23 wherein said dehydration catalyst is acidic.

30. A process for reacting methylal with a saturated monocarboxylic acid ester to form an ester of an alpha, beta-ethylenically unsaturated acid which comprises:

(a) providing an admixture comprising methylal and at least one ester of a saturated monocarboxylic acid;

(b) contacting said admixture, in the vapor phase in at least one reaction zone, with a dehydration catalyst in a manner and under conditions sufficient to react said admixture and produce an effluent mixture comprising water, the alpha, beta-ethylenically unsaturated acid ester condensation product of methylal and said saturated monocarboxylic acid ester, the corresponding free acid of said alpha, beta-ethylenically unsaturated acid ester, unreacted saturated monocarboxylic acid ester, an organic alcohol and free acid of said saturated monocarboxylic acid ester both derived from hydrolysis of said saturated acid ester, at least one ether by-product selected from the group consisting of dimethyl ether and an ether derived from said organic alcohol, and optionally unreacted methylal, and formaldehyde derived from methylal;

(c) separating and recovering said ether by-product from the effluent mixture and recycling at least a portion of said ether by-product for combination with said admixture of step (a) and reaction in accordance with step (b), the amount of said ether recycled being sufficient to increase the alpha, beta-ethylenically unsaturated acid ester: alpha, beta-ethylenically unsaturated acid mole ratio in the effluent mixture relative to the absence of said ether by-product; said effluent mixture after recovery of the ether by-product therefrom being designated residual effluent mixture;

(d) recovering at least a portion of the saturated monocarboxylic acid present in the residual effluent mixture;

(e) recovering at least a portion of the alpha, beta-ethylenically unsaturated acid from the residual effluent mixture;

(f) recovering at least a portion of the alpha, beta-ethylenically unsaturated acid ester and water from the residual effluent mixture;

(g) recovering of at least a portion of the unreacted saturated monocarboxylic acid ester from the residual effluent mixture;

(h) admixing and reacting in at least one reaction zone separate from the reaction zone of step (b) at least a portion of the ether by-product recovered in accordance with step (c) with: (i) at least a portion of the saturated monocarboxylic acid recovered in accordance with step (d) and, (ii) optionally, at least a portion of the alpha, beta-ethylenically unsaturated acid recovered in accordance with step (e), in a manner and under conditions sufficient to convert at least a portion of reactant (i) to its corresponding saturated acid ester and reactant (ii) when present in said admixture, to its corresponding unsaturated acid ester, and recovering at least a portion of the esters so produced:

(i) recycling at least a portion of the saturated monocarboxylic acid ester recovered in accordance with one or more of steps (g) and (h) to the admixture of step (a) for reaction in accordance with step (b); and

(j) optionally, recovering at least a portion of the methylal and formaldehyde when present in the residual effluent mixture and recycling at least a portion of said methylal and formaldehyde for combination with the admixture of step (c) and reaction in accordance with step (b).

31. The process of Claim 30 wherein all of the saturated monocarboxylic acid ester admixed in accordance with step (a) is produced by modifying step (h) to include as an additional reactant to be reacted with said ether by-product, fresh saturated monocarboxylic acid introduced from a source external to the residual effluent mixture.

32. The process of Claim 30 wherein said saturated monocarboxylic acid ester is represented by the structural formula:

$$R—CH_2—COOR'$$

wherein R and R' are hydrocarbyl groups independently selected from the group consisting of alkyl, aryl, aralkyl and alkaryl, and said ether by-product comprises at least one ether represented by the structural formula:

$$R'—O—R'$$

wherein R' is as described above.

33. The process of Claim 32 wherein R and R' represent $C_1$ to $C_5$ alkyl.

34. The process of Claim 30 wherein the saturated monocarboxylic acid ester is methyl propionate, the corresponding alpha, beta-ethylenically unsaturated acid ester is methyl methacrylate, and the ether by-product is dimethyl ether.

35. The process of Claim 30 wherein at least a portion of the water recovered in accordance with step (f) is recycled to the reaction zone of step (h) in a manner and under conditions sufficient to control the amount of by-product ether admixed with said acid reactants by hydrolysis of said ether.

36. The process of Claim 30 wherein the dehydration catalyst is acidic.

**Patentansprüche**

1. Verfahren zur Umsetzung einer dampfförmigen Mischung, umfassend (a) mindestens ein Mitglied, ausgewählt aus der Gruppe bestehend aus gesättiger Carbonsäure, und einem Ester dieser gesättigten Carbonsäure und (b) mindestens ein Mitglied, ausgewählt aus der Gruppe bestehend aus Formaldehyd und einem Derivat von Formaldehyd in Gegenwart eines Dehydratisierungskatalysators zur Herstellung eines Produkts, umfassend mindestens ein Mitglied, ausgewählt aus der Gruppe bestehend aus α,β-ethylenisch ungesättigter Säure und einem Ester dieser ungesättigten Säure durch eine Kondensationsreaktion, dadurch gekennzeichnet, dass mindestens ein Ether in die genannte dampfförmige Mischung in einer Weise und unter Bedingungen eingeleitet wird, die ausreichen, um eine Erhöhung in der proportionalen Verteilung dieses α,β-ethylenisch ungesättigten Säureesters in dem genannten Produkt im Vergleich zur Abwesenheit dieser Etherzuführung zu bewirken, wobei der genannte Ether dadurch charakterisiert ist, dass er die Fähigkeit hat, (a) den genannten Carbonsäure-Reaktionsteilnehmer in seinen entsprechenden Ester durch Reaktion mit ihm zu überführen, (b) die genannte α,β-ethylenisch ungesättigte Säure in ihren entsprechenden Ester durch Umsetzung mit ihr zu überführen, und gegebenenfalls (c) unter sauren Reaktionsbedingungen in Gegenwart von Wasser zu seinem entsprechenden Alkohol zu hydrolysieren.

2. Verfahren nach Anspruch 1, dadurch gekennzeichnet, dass der Dehydratisierungskatalysator basisch ist.

3. Verfahren nach Anspruch 1, dadurch gekennzeichnet, dass der Dehydratisierungskatalysator sauer ist.

4. Verfahren nach Anspruch 1, dadurch gekennzeichnet, dass der genannte Ether als Nebenprodukt während der genannten Kondensationsreaktion gebildet wird.

5. Verfahren nach Anspruch 4, dadurch gekennzeichnet, dass das genannte Ether-Nebenprodukt von dem Kondensationsreaktionsprodukt abgetrennt und zur Einführung in die genannte dampfförmige Mischung zurückgeführt wird.

6. Verfahren nach Anspruch 1, dadurch gekennzeichnet, dass die dampfförmige Mischung zumindest eine Verbindung der Strukturformel

$$R—CH_2—COOR'$$

umfasst, worin R und R' unabhängig voneinander ausgewählt sind aus der Gruppe bestehend aus Wasserstoff, Alkyl, Aryl, Aralkyl und Alkaryl.

7. Verfahren nach Anspruch 6, dadurch gekennzeichnet, dass R nicht Wasserstoff ist.

8. Verfahren nach Anspruch 6, dadurch gekennzeichnet, dass R und R' unabhängig voneinander $C_1$-bis $C_5$-Alkyl bedeuten.

9. Verfahren nach Anspruch 6, dadurch gekennzeichnet, dass der genannte Ether die Strukturformel

$$R'—O—R'$$

aufweist, worin R' die im Zusammenhang mit der Strukturformel des Anspruchs 6 beschriebene Bedeutung hat, jedoch mit der Ausnahme, dass R' nicht Wasserstoff ist.

10. Verfahren nach Anspruch 1, dadurch gekennzeichnet, dass die dampfförmige Mischung mindestens eine Verbindung, ausgewählt aus der Gruppe bestehend aus Formaldehyd, Methylal und dem Hemiacetal von Formaldehyd umfasst.

11. Verfahren nach Anspruch 1, dadurch gekennzeichnet, dass die dampfförmige Mischung (a) mindestens einen aus der Gruppe bestehend aus Propionsäure und Methylpropionat ausgewählten Reaktionsteilnehmer und (b) mindestens einen aus der Gruppe, bestehend aus Formaldehyd, Methylal und dem Hemiacetal von Formaldehyd ausgewählten Reaktionsteilnehmer umfasst und dass der in die dampfförmige Reaktionsmischung eingeführte Ether Dimethylether ist.

12. Verfahren nach einem der Ansprüche 1 bis 11, dadurch gekennzeichnet, dass der Dehydratisierungskatalysator sauer ist und durch das Verfahren hergestellt wird umfassend

(1) die Umsetzung einer Mischung von mindestens einem Metallkohlenwasserstoffoxid I, mindestens einem Metallkohlenwasserstoffoxid II und mindestens einer sauren Phosphor und Sauerstoff enthaltenden Verbindung mit Wasser in Gegenwart von mindestens einem flüssigen organischen Medium, enthaltend mindestens 50 Gew.-%, bezogen auf das Gewicht dieses Mediums, mindestens eine Mitgliedes ausgewählt aus der Gruppe bestehend aus organischem Aldehyd, organischem Keton und organischem Ether, wobei diese Reaktion in einer Weise geführt wird, die ausreicht, um (a) den Kontakt der Metallkohlenwasserstoffoxide I und II mit Wasser vor dem Kontakt der Metallkohlenwasserstoffoxide I und II mit der sauren Phosphor und Sauerstoff enthaltenden Verbindung zu vermeiden und (b) die Vorläuferverbindung des Katalysators zu bilden,

(2) die Abtrennung dieser Katalysatorvorläuferzusammensetzung von der genannten Reaktionsmischung,

(3) Calcinieren der genannten Katalysatorvorläuferzusammensetzung zur Bildung der Katalysatorzusammensetzung, worin

(i) das Metall $M^1$ des genannten Metallkohlenwasserstoffoxids I ausgewählt ist aus mindestens einem der Mitglieder der Gruppe bestehend aus Al, Ga, In und Tl und

(ii) das Metall $M^2$ des Metallkohlenwasserstoffoxids II ausgewählt ist aus mindestens einem Mitglied der Gruppe bestehend aus Si, SN, Zr und Ge.

13. Verfahren nach Anspruch 12, dadurch gekennzeichnet, dass in dem genannten Verfahren zur Herstellung des Katalysators das Metallkohlenwasserstoffoxid I mindestens ein Aluminiumalkoxid, das Metallkohlenwasserstoffoxid II mindestens ein Siliciumalkoxid und die saure Phosphor-Sauerstoff-Verbindung Phosphorsäure ist.

14. Verfahren nach Anspruch 12, dadurch gekennzeichnet, dass die dampfförmige Mischung zusätzlich mindestens eine aromatische Verbindung zur Reduzierung der Koksbildung des genannten Katalysators umfasst.

15. Verfahren zur Herstellung einer α,β-ethylenisch ungesättigten Säure und deren Ester, umfassend

(a) zur Verfügungsstellung einer Mischung umfassend wenigstens eine gesättigte Monocarbonsäure, den Methylester dieser gesättigten Carbonsäure, wässriges Formaldehyd und Methanol,

(b) Umsetzung dieser Mischung in der Dampfphase und in wenigstens einer Reaktionszone in Gegenwart eines Dehydratisierungskatalysators in einer Weise und unter Bedingungen, die ausreichen, um eine ausfliessende Mischung enthaltend Wasser, die entsprechende α,β-ethylenisch ungesättigte Säure und deren Methylesterderivat, Dimethylether, nichtumgesetzte gesättigte Monocarbonsäure und den nichtumgesetzten Methylester dieser gesättigten Monocarbonsäure zu bilden,

(c) Abtrennen und Wiedergewinnung des Dimethylethers aus dieser ausfliessenden Mischung und Rückführung mindestens eines Teils des zurückgewonnenen Dimethylethers in mindestens eine der genannten Reaktionszonen zur Kombination mit der Mischung aus Stufe (a) in einer Weise und in einer Menge, die ausreicht, um zumindest einen Teil der gesättigten Monocarbonsäure in dieser Mischung in den entsprechenden Methylester zu überführen, wobei diese ausfliessende Mischung nach der Rückgewinnung des Dimethylethers daraus als restliche ausfliessende Mischung bezeichnet wird,

(d) Zurückgewinnung mindestens eines Teils der nicht umgesetzten gesättigten Monocarbonsäure in der restlichen ausfliessenden Mischung und Umsetzung mindestens eines Teils dieser zurückgewonnenen gesättigten Säure mit mindestens einem Teil des gemäss Schritt (c) zurückgewonnenen Dimethylethers zur Bildung des entsprechenden Methylesterderivats dieser Säure und Gewinnung dieses Esters,

(e) Zurückgewinnung mindestens eines Teils des nicht umgesetzten Methylesters der gesättigten Monocarbonsäure in der restlichen ausfliessenden Mischung,

(f) Umsetzung mindestens eines Teils des gesättigten Monocarbonsäuremethylesters, gewonnen nach einem oder mehreren der Schritte (d) und (e), mit Formaldehyd in Gegenwart von einem Dehydratisierungskatalysators, Methanol und in der Dampfphase zur Herstellung des entsprechenden α,β-ethylenisch ungesättigten Methylesters davon,

(g) Gewinnung der α,β-ethylenisch ungesättigten Säure und deren Methylesterderivat, hergestellt entsprechend den Schritten (a) bis (f).

16. Verfahren nach Anspruch 15, dadurch gekennzeichnet, dass die Reaktion der gesättigten Säure mit Dimethylether gemäss Schritt (d) in der Weise erfolgt, dass die genannte gesättigte Säure zurückgeführt wird und ausreichend Dimethylether gemäss Schritt (c) zur Vereinigung mit der genannten Mischung des Schritts (a) und Reaktion in der genannten Reaktionszone gemäss Schritt (b) gewonnen wird.

17. Verfahren nach Anspruch 15, dadurch gekennzeichnet, dass die Reaktion gemäss Schritt (f) in der Weise erfolgt, dass mindestens ein Teil des gesättigten Esters, der gemäss einem oder mehreren der Schritte (d) und (e) gewonnen wird, zur Kombination mit der Mischung des Schritts (a) und zur Reaktion in der genannten Reaktionszone des Schrittes (b) zurückgeführt wird.

18. Verfahren nach Anspruch 15, dadurch gekennzeichnet, dass mindestens ein Teil der α,β-ethylenisch ungesättigten Säure, die gemäss Schritt (g) zurückgewonnen wird, mit einer ausreichenden Menge Dimethylether umgesetzt wird, um den entsprechenden Methylester der genannten ungesättigten Säure zu bilden.

19. Verfahren nach Anspruch 18, dadurch gekennzeichnet, dass die Reaktion durch Rückführen der genannten ungesättigten Säure und ausreichend Dimethylether, gewonnen gemäss Schritt (c), zur Kombination mit der Mischung des Schritts (a) und der Reaktion in der Reaktionszone des Schritts (b) zurückgeführt wird.

20. Verfahren nach Anspruch 15, dadurch gekennzeichnet, dass die gesättigte Monocarbonsäure Propionsäure ist.

21. Verfahren nach Anspruch 15, dadurch gekennzeichnet, dass der Dehydratisierungskatalysator sauer ist.

22. Verfahren nach Anspruch 15, dadurch gekennzeichnet, dass ausreichend Dimethylether in die Reaktionszone des Schritts (b) eingeführt wird, um auch die Hydrolyse des genannten Ethers zu Methanol zu bewirken, um mindestens einen Teil des in der Reaktionszone anwesenden Wassers herauszuspülen.

23. Verfahren zur Herstellung von α,β-ethylenisch ungesättigter Säure und deren Ester, umfassend:

(a) Herstellen einer Mischung bestehend aus wenigstens einer gesättigten Monocarbonsäure und Methylal,

(b) Zusammenbringen dieser Mischung in der Gasphase in wenigstens einer Reaktionszone mit einerm sauren Dehydratisierungskatalysator in einer Weise und unter Bedingungen, die ausreichen, um diese

Mischung umzusetzen und eine ausfliessende Mischung herzustellen, die Wasser, das α,β-ethylenisch ungesättigte Säurekondensationsprodukt von Methylal und der genannten gesättigten Säure, das entsprechende Methylesterderivat der genannten ungesättigten Säure, Dimethylether, nichtumgesetzte gesättigte Monocarbonsäuren und gegebenenfalls dessen Methylesterderivat enthält,

(c) Abtrennen und Rückgewinnen des Dimethylethers aus dieser ausfliessenden Mischung und Rückführung mindestens eines Teils dieses Ether zu mindestens einer der genannten Reaktionszonen zur Kombination mit der genannten Mischung des Schritts (a) in einer Menge, die ausreicht, um mindestens einen Teil der in dieser Mischung vorhandenen Monocarbonsäure in den entsprechenden Methylester zu überführen, wobei diese ausfliessende Mischung nach Abtrennen des Dimethylethers als restliche ausfliessende Mischung bezeichnet wird,

(d) Rückgewinnung mindestens eines Teils der nichtumgesetzten gesättigten Monocarbonsäure in der restlichen ausfliessenden Mischung und Umsetzung mindestens eines Teils dieser rückgewonnenen gesättigten Säure mit mindestens einem Teil des gemäss Schritt (c) zurückgewonnenen Dimethylethers zur Bildung des entsprechenden methylesterderivats dieser Säure und Gewinnen dieses Esters,

(e) gegebenenfalls Rückgewinnen wenigstens eines Teils des Methylesters der gesättigten Monocarbonsäure, vorhanden in der restlichen ausfliessenden Mischung,

(f) Umsetzung wenigstens eines Teils des nach einem oder mehreren der Schritte (d) und (e) zurückgewonnenen gesättigten Monocarbonsäuremethylesters mit Methylal in Gegenwart eines Dehydratisierungskatalysators in der Dampfphase zur Herstellung des entsprechenden α,β-ethylenisch ungesättigten Säureesterkondensationsprodukts davon,

(g) Gewinnung der α,β-ethylenisch ungesättigten Säure und deren Methylesterderivat, hergestellt gemäss den Schritten (a) bis (f).

24. Verfahren nach Anspruch 23, dadurch gekennzeichnet, dass die Umsetzung der genannten gesättigten Säure mit Dimethylether gemäss Schritt (d) durch Zurückführen der genannten gesättigten Säure und ausreichend Dimethylether, gewonnen gemäss Schritt (c), zur Kombination mit der genannten Mischung des Schritts (a) und Umsetzung in der Reaktionszone des Schrittes (b) erfolgt.

25. Verfahren nach Anspruch 23, dadurch gekennzeichnet, dass die Umsetzung gemäss Schritt (f) durch Rückführen des nach einem oder mehreren der Schritte (d) und (e) gewonnen gesättigten Esters zur Kombination mit der Mischung des Schrittes (a) und zur Umsetzung in der Reaktionszone des Schrittes (b) erfolgt.

26. Verfahren nach Anspruch 23, dadurch gekennzeichnet, dass zumindest ein Teil der gemäss Schritt (g) zurückgewonnenen α,β-ethylenisch ungesättigten Säure mit genügend Dimethylether umgesetzt wird, um den entsprechenden Methylester der genannten ungesättigten Säure zu bilden.

27. Verfahren nach Anspruch 26, dadurch gekennzeichnet, dass die Reaktion durch Rückführung der genannten ungesättigten Säure und genügender Menge gemäss dem Schritt (c) gewonnenen Dimethylethers zur Kombination mit der Mischung des Schritts (a) und der Umsetzung in der Reaktionszone des Schritts (b) durchgeführt wird.

28. Verfahren nach Anspruch 23, dadurch gekennzeichnet, dass die gesättigte Monocarbonsäure Propionsäure ist.

29. Verfahren nach Anspruch 23, dadurch gekennzeichnet, dass der Dehydratisierungskatalysator sauer ist.

30. Verfahren zur Umsetzung von Methylal mit einem gesättigten Monocarbonsäureester zur Bildung eines Esters einer α,β-ethylenisch ungesättigten Säure, das umfasst:

(a) Bereitstellung einer Mischung enthaltend Methylaldehyd und wenigstens einen Ester einer gesättigten Monocarbonsäure,

(b) Zusammenbringen dieser Mischung in der Dampfphase in mindestens einer Reaktionszone mit einem Dehydratisierungskatalysator in einer Weise und unter Bedingungen, die ausreichen, um diese Mischung umzusetzten und eine ausfliessende Mischung herzustellen, die Wasser, das α,β-ethylenisch ungesättigte Säureester-Kondensationsprodukt von Methylal und dem gennanten gesättigten Monocarbonsäureester, die entsprechende freie Säure des genannten α,β-ethylenisch ungesättigten Säureesters, nichtumgesetzten gesättigten Monocarbonsäureester, einen organischen Alkohol und freie Säure des genannten gesättigten Monocarbonsäureesters, beide erhalten durch die Hydrolyse des genannten gesättigten Säureesters, wenigstens ein Ether-Nebenprodukt, ausgewählt aus der Gruppe bestehend aus Dimethylether und einem von dem genannten organischen Alkohol abgeleiteten Ether, und gegebenenfalls nichtumgesetztes Methylal und von Methylal abgeleitetes Formaldehyd enthält,

(c) Abtrennen und Gewinnen des genannten Ether-Nebenprodukts aus der ausfliessenden Mischung und Rückführung mindestens eines Teils dieses Ether-Nebenprodukts zur Kombination mit der genannten Mischung des Schritts (a) und Umsetzung gemäss Schritt (b), wobei die Menge dieses zurückgeführten Ethers ausreicht zur Vergrösserung des Molverhältnisses des α,β-ethylenisch ungesättigten Säureesters zu α,β-ethylenisch ungesättigter Säure in der ausfliessenden Mischung im Vergleich zur Abwesenheit dieses Ether-Nebenprodukts, wobei die ausfliessende Mischung nach der Rückgewinnung des Ether-Nebenprodukts daraus als restliche ausfliessende Mischung bezeichnet wird,

(d) Rückgewinnung wenigstens eines Teils der gesättigten Monocarbonsäure in der restlichen ausfliessenden Mischung,

(e) Rückgewinnung wenigstens eines Teils der α,β-ethylenisch ungesättigten Säure aus der restlichen ausfliessenden Mischung,

(f) Gewinnung wenigstens eines Teils des α,β-ethylenisch ungesättigten Säureesters und Wassers aus der restlichen ausfliessenden Mischung,

(g) Rückgewinnung wenigstens eines Teils des nichtumgesetzten gesättigten Monocarbonsäureesters aus der restlichen ausfliessenden Mischung,

(h) Mischen und Umsetzen in wenigstens einer Reaktionszone getrennt von der Reaktionszone des Schrittes (b) von wenigstens einem Teil des gemäss Schritt (c) zurückgewonnenen Teils des Ether-Nebenprodukts mit (i) wenigstens einem Teil der gemäss Schritt (d) zurückgewonnenen gesättigten Monocarbonsäure und (ii) gegebenenfalls wenigstens einem Teil der α,β-ethylenisch ungesättigten, gemäss Schritt (e) zurückgewonnenen Säure in einer Weise und unter Bedingungen, die ausreichen, um zumindestens einen Teil des Reaktionsteilnehmer (i) in den entsprechenden gesättigten Säureester und des Reaktionsteilnehmers (ii), wenn in der genannten Mischung vorhanden, in den entsprechenden ungesättigten Säureester zu überführen und wenigstens einen Teil der so hergestellten Ester zu gewinnen,

(i) Zurückführung wenigstens eines Teils gemäss einem oder mehreren der Schritt (g) und (h) zurückgewonnenen gesättigten Monocarbonsäureesters zu der Mischung des Schritts (a) zur Umsetzung entsprechend Schritt (b) und

(j) gegebenenfalls Zurückgewinnung eines Teils des in der restlichen ausfliessenden Mischung vorhandenen Methylal und Formaldehyds und Rückführung wenigstens eines Teil dieses Methylal und Formaldehyds zur Kombination mit der Mischung des Schrittes (c) und Umsetzung gemäss Schritt (b).

31. Verfahren nach Anspruch 30, dadurch gekennzeichnet, dass der gemäss Schritt (a) zugemischte gesättigte Monocarbonsäureester hergestellt wird durch Abwandlung des Schrittes (h) durch Einbeziehung von frischer gesättigter Monocarbonsäure, zugeführt aus einer externen Quelle zu der restlichen ausfliessenden Mischung als zusätzlicher Reaktionsteilnehmer zur Umsetzung mit dem genannten Ether-Nebenprodukt.

32. Verfahren nach Anspruch 30, dadurch gekennzeichnet, dass der genannte gesättigte Monocarbonsäureester die Strukturformel

$$R—CH_2—COOR'$$

aufweist, worin R und R' unabhängig voneinander Kohlenwasserstoffgruppen, ausgewählt aus der Gruppe bestehend aus Alkyl, Aryl, Aralkyl und Alkaryl, sind und das genannte Ether-Nebenprodukt mindestens einen Ether der Strukturformel

$$R'—O—R'$$

worin R' die vorstehend genannten Bedeutungen hat, ist.

33. Verfahren nach Anspruch 32, dadurch gekennzeichnet, dass R und R' $C_1$- bis $C_5$-Alkylreste sind.

34. Verfahren nach Anspruch 30, dadurch gekennzeichnet, dass der gesättigte Monocarbonsäureester Methylpropionat, der entsprechende α,β-ethylenisch ungesättigte Säureester Methylmethacrylat und das Ether-Nebenprodukt Dimethylether sind.

35. Verfahren nach Anspruch 30, dadurch gekennzeichnet, dass wenigstens ein Teil des gemäss Schritt (f) zurückgewonnenen Wassers in die Reaktionszone des Schrittes (h) in einer Weise und unter Bedingungen zurückgeführt wird, der ausreicht, durch Hydrolyse des Nebenproduktethers die Menge des mit den genannten sauren Reaktionsteilnehmern gemischten Nebenprodukt-Ethers zu kontrollieren.

36. Verfahren nach Anspruch 30, dadurch gekennzeichnet, dass der Dehydratisierungskatalysator sauer ist.

**Revendications**

1. Procédé de réaction d'un mélange à l'état de vapeur, comprenant (a) au moins un membre du groupe constitué d'un acide carboxylique saturé et d'un ester de cet acide carboxylique et (b) au moins un membre du groupe constitué du formaldéhyde et d'un dérivé du formaldéhyde, en présence d'un catalyseur de déshydratation pour former, par une réaction de condensation, un produit comprenant au moins un membre choisi dans le groupe constitué d'un acide à non-saturation alpha, bêta-éthylénique et d'un ester dudit acide non sauré, le perfectionnement consistant à introduire au moins un éther dans ledit mélange à l'état de vapeur d'une manière et dans des conditions qui suffisent à provoquer une élévation de la distribution proportionnelle dudit ester d'acide à non-saturation alpha, bêta-éthylénique dans ledit produit par rapport à l'absence d'introduction dudit éther; ce dernier étant caractérisé par son aptitude (a) à convertir l'acide carboxylique réactionnel en son ester correspondant par réaction avec lui; (b) à convertir ledit acide à non-saturation alpha, bêta-éthylénique en son ester correspondant par réaction avec lui; et à titre facultatif (c) à l'hydrolyser dans des conditions réactionnelles acides en présence d'eau en son alcool correspondant.

2. Procédé suivant la revendication 1, dans lequel le catalyseur de déshydratation est basique.

3. Procédé suivant la revendication 1, dans lequel le catalyseur de déshydratation est acide.

4. Procédé suivant la revendication 1, dans lequel l'éther est formé comme sous-produit pendant ladite réaction de condensation.

5. Procédé suivant la revendication 4, dans lequel l'éther formé comme sous-produit est séparé du produit de réaction de condensation et recyclé en vue de son introduction dans ledit mélange à l'état de vapeur.

6. Procédé suivant la revendication 1, dans lequel le mélange à l'état de vapeur comprend au moins un composé représenté par la formule structurale:

$$R—CH_2—COOR'$$

dans laquelle R et R' sont choisis indépendamment dans le groupe comprenant l'hydrogène et des radicaux alkyle, aryle, aralkyle et alkaryle.

7. Procédé suivant la revendication 6, dans lequel R est autre chose que l'hydrogène.

8. Procédé suivant la revendication 6, dans lequel R et R' représentent indépendamment des groupes alkyle en $C_1$ à $C_5$.

9. Procédé suivant la revendication 6, dans lequel l'éther est représenté par la formule structurale:

$$R'—O—R'$$

dans laquelle R' est tel que défini conjointement avec la formule structurale de la revendication 6 dans laquelle R' est autre chose que de l'hydrogène.

10. Procédé suivant la revendication 1, dans lequel le mélange à l'état de vapeur comprend au moins un composé choisi dans le groupe constitué du formaldéhyde, du méthylal et de l'hémi-acétal du formaldéhyde.

11. Procédé suivant la revendication 1, dans lequel le mélange à l'état de vapeur comprend (a) au moins un corps réactionnel choisi dans le groupe comprenant l'acide propionique et le propionate de méthyle; et (b) au moins un corps réactionnel choisi dans le groupe comprenant le formaldéhyde, le méthylal et l'hémi-acétal du formaldéhyde; et ledit éther qui est introduit dans ledit mélange à l'état de vapeur est l'éther de diméthyle.

12. Procédé suivant l'une quelconque des revendications 1 à 11, dans lequel le catalyseur de déshydratation est acide et est préparé par le procédé qui consiste:

(1) à faire réagir en mélange au moins un Hydrocarboxyde Métallique I, au moins un Hydrocarboxyde Métallique II, au moins un composé acide contenant du phosphore et de l'oxygène, et de l'eau, en présence d'au moins un milieu organique liquide comprenant au moins 50% en poids, sur la base dudit milieu, d'au moins un représentant du groupe comprenant un aldéhyde organique, une cétone organique et un éther organique, ladite réaction étant conduite d'une manière suffisante (a) pour éviter le contact des Hydrocarboxydes Métalliques I et II avec l'eau avant le contact des Hydrocarboxydes Métalliques I et II avec le composé acide contenant du phosphore et de l'oxygène, et (b) pour former une composition de précurseur de catalyseur;

(2) à séparer la composition de précurseur de catalyseur dudit mélange réactionnel;

(3) à calciner la composition de précurseur de catalyseur pour former ladite composition de catalyseur; procédé dans lequel:

(i) le métal $M^1$ de l'Hydrocarboxyde Métallique I est au moins un représentant du groupe comprenant l'aluminium, le gallium, l'indium et le tallium; et

(ii) le métal $M^2$ de l'Hydrocarboxyde Métallique II est au moins un représentant du groupe comprenant le silicium, l'étain, le zirconium et le germanium.

13. Procédé suivant la revendication 12, dans l'opération de préparation du catalyseur duquel, l'Hydrocarboxyde Métallique I est au moins un alcoxyde d'aluminium, l'Hydrocarboxyde Métallique II est au moins un alcoxyde de silicium et le composé acide contenant du phosphore et de l'oxygène est l'acide phosphorique.

14. Procédé suivant la revendication 12, dans lequel le mélange à l'état de vapeur comprend en outre au moins un composé aromatique pour réduire la formation de coke sur ledit catalyseur.

15. Procédé de production d'un acide à non-saturation alpha, bêta-éthylénique et d'un ester de cet acide, qui consiste:

(a) à préparer un mélange comprenant au moins un acide monocarboxylique sauré, l'ester méthylique dudit acide carboxylique sauré, du formaldéhyde aqueux et du méthanol;

(b) à faire réagir ledit mélange en phase vapeur et dans au moins une zone de réaction, en présence d'un catalyseur de déshydratation, d'une manière et dans des conditions suffisantes pour former un effluent comprenant en mélange de l'eau, l'acide à non-saturation alpha, bêta-éthylénique correspondant et l'ester méthylique qui en est dérivé, de l'éther de diméthyle, de l'acide monocarboxylique sauré n'ayant pas réagi et l'ester méthylique dudit acide monocarboxylique sauré n'ayant pas réagi;

(c) à séparer et recueillir l'éther de diméthyle dudit mélange effluent et à recycler au moins une portion de l'éther de diméthyle recueilli dans au moins l'une desdites zones de réaction en vue de réunir cette portion avec ledit mélange de l'étape (a) d'une manière et en une quantité suffisantes pour convertir au moins une portion de l'acide monocarboxylique sauré présent dans ledit mélange en son ester méthylique

correspondant; ledit mélange d'effluent, après que l'éther de diméthyle en a été recueilli, étant appelé mélange effluent résiduel;

(d) à recueillir au moins une portion de l'acide monocarboxylique sauré n'ayant pas réagi présent dans le mélange effluent résiduel et à faire réagir au moins une portion dudit acide saturé recueilli avec au moins une portion dudit éther de diméthyle recueilli conformément à l'étape (c) pour former l'ester méthylique correspondant dudit acide et à recueillir ledit ester;

(e) à receuillir au moins une portion de l'ester méthylique de l'acide monocarboxylique saturé n'ayant pas réagi, présent dans le mélange effluent résiduel;

(f) à faire réagir au moins une portion de l'ester méthylique d'acide monocarboxylique saturé, recueilli conformément à une ou plusieurs des étapes (d) et (e), avec du formaldéhyde en présence d'un catalyseur de déshydratation, de méthanol, et en phase vapeur pour produire l'ester méthylique d'acide à non-saturation alpha, bêta-éthylénique correspondant;

(g) à recueillir l'acide à non-saturation alpha, bêta-éthylénique et son ester méthylique produits conformément aux étapes (a) à (f).

16. Procédé suivant la revendication 15, dans lequel la réaction dudit acide saturé avec l'éther de diméthyle conformément à l'étape (d) est conduite par recyclage de l'acide sauré et d'une quantité suffisante d'éther diméthylique recueilli conformément à l'étape (c) pour la réunion avec ledit mélange de l'étape (a) et la réaction dans ladite zone de réaction de l'étape (b).

17. Procédé suivant la revendication 15, dans lequel la réaction conformément à l'étape (f) est conduite par recyclage d'au moins une portion de l'ester saturé recueilli conformément à une ou plusieurs des étapes (d) et (e) en vue de sa réunion avec le mélange de l'étape (a) et de la réaction dans ladite zone réactionnelle de l'étape (b).

18. Procédé suivant la revendication 15, dans lequel une portion au moins de l'acide à non-saturation alpha, bêta-éthylénique recueilli conformément à l'étape (g) est amenée à réagir avec une quantité suffisante d'éther de diméthyle pour former l'ester méthylique correspondant dudit acide non saturé.

19. Procédé suivant la revendication 18, dans lequel ladite réaction est conduite par recyclage dudit acide non saturé et d'une quantité suffisante d'éther diméthylique recueilli conformément à l'étape (c) pour la réunion avec le mélange de l'étape (a) et la réaction dans la zone de réaction de l'étape (b).

20. Procédé suivant la revendication 15, dans lequel ledit acide monocarboxylique saturé est l'acide propionique.

21. Procédé suivant la revendication 15, dans lequel le catalyseur de déshydratation est acide.

22. Procédé suivant la revendication 15, dans lequel on introduit suffisamment d'éther de diméthyle dans la zone de réaction de l'étape (b) pour provoquer également l'hydrolyse dudit éther en méthanol de manière à fixer une portion au moins de l'eau présente dans ladite zone de réaction.

23. Procédé de production d'acide à non-saturation alpha, bêta-éthylénique et d'un ester de cet acide, qui consiste:

(a) à préparer un mélange comprenant au moins un acide monocarboxylique sauré et du méthylal;

(b) à faire entrer ledit mélange, en phase vapeur dans au moins une zone de réaction, en contact avec un catalyseur acide de déshydratation d'une manière et dans des conditions suffisantes pour faire réagir ledit mélange et pour produire un mélange effluent, comprenant de l'eau, le produit de condensation d'acide à non-saturation alpha, bêta-éthylénique du méthylal et ledit acide saturé, l'ester méthylique correspondant dudit acide non saturé, de l'éther de diméthyle, de l'acide monocarboxylique saturé n'ayant pas réagi et, à titre facultatif, l'ester méthylique dérivé de cet acide;

(c) à séparer et recueillir l'éther de diméthyle dudit mélange effluent et à recycler au moins une portion de cet éther dans au moins l'une desdites zones de réaction en vue de sa réunion avec le mélange de l'étape (a) en une quantité suffisante pour convertir au moins une portion dudit acide monocarboxylique sauré présent dans ledit mélange en son ester méthylique correspondant; ledit mélange effluent, après que l'éther de diméthyle en a été recueilli, étant appelé mélange effluent résiduel;

(d) à receuillir au moins une portion de l'acide monocarboxylique saturé n'ayant pas réagi, présent dans le mélange effluent résiduel, et à faire réagir au moins une portion dudit acide saturé recueilli avec au moins une portion de l'éther diméthylique recueilli conformément à l'étape (c), pour former l'ester méthylique correspondant dudit acide, et à recueillir ledit ester;

(e) à titre facultatif, à recueillir au moins une portion de l'ester méthylique de l'acide monocarboxylique saturé présent dans le mélange effluent résiduel;

(f) à faire réagir au moins une portion de l'ester méthylique d'acide monocarboxylique saturé recueilli conformément à une ou plusieurs des étapes (d) et (e) avec du méthylal en présence d'un catalyseur de déshydratation en phase vapeur pour former le produit de condensation ester d'acide à non-saturation alpha, bêta-éthylénique correspondant;

(g) à recueillir l'acide à non-saturation alpha, béta-éthylénique et son ester méthylique produit conformément aux étapes (a) à (f).

24. Procédé suivant la revendication 23, dans lequel la réaction de l'acide saturé avec l'éther de diméthyle conformément à l'étape (d) est conduite par recyclage dudit acide saturé et d'une quantité suffisante de l'éther de diméthyle recueilli conformément à l'étape (c) pour sa réunion avec ledit mélange de l'étape (a), et sa réaction dans ladite zone de réaction de l'étape (b).

25. Procédé suivant la revendication 23, dans lequel la réaction conformément à l'étape (f) est conduite

par recyclage de l'ester saturé recueilli conformément à une ou plusieurs des étapes (d) et (e) pour sa réunion avec le mélange de l'étape (a) et sa réaction dans ladite zone de réaction de l'étape (b).

26. Procédé suivant la revendication 23, dans lequel une portion d'au moins de l'acide à non-saturation alpha, bêta-éthylénique recueilli conformément à l'étape (g) est amenée à réagir avec une quantité suffisante d'éther de diméthyle pour former l'ester méthylique correspondant dudit acide non saturé.

27. Procédé suivant la revendication 26, dans lequel la réaction est conduite par recyclage de l'acide non saturé et d'une quantité suffisante d'éther de diméthyle recueilli conformément à l'étape (c) pour sa réunion avec le mélange de l'étape (a) et sa réaction dans la zone de réaction de l'étape (b).

28. Procédé suivant la revendication 23, dans lequel l'acide monocarboxylique saturé est l'acide propionique.

29. Procédé suivant la revendication 23, dans lequel le catalyseur de déshydratation est acide.

30. Procédé de réaction du méthylal avec un ester d'acide monocarboxylique saturé pour former un ester d'un acide à non-saturation alpha, bêta-éthylénique, qui consiste:

(a) à préparer un mélange comprenant du méthylal et au moins un ester d'un acide monocarboxylique saturé;

(b) à faire enter ledit mélange, en phase vapeur dans au moins une zone de réaction, en contact avec un catalyseur de déshydratation d'une manière et dans des conditions suffisantes pour faire réagir ledit mélange et pour produire un mélange effluent comprenant de l'eau, l'ester d'acide à non-saturation alpha, bêta-éthylénique formé comme produit de condensation entre le méthylal et ledit ester d'acide monocarboxylique saturé, l'acide libre correspondant dudit ester d'acide à non-saturation alpha, bêta-éthylénique, de l'ester d'acide monocarboxylique saturé n'ayant pas réagi, un alcool organique et l'acide libre dudit ester d'acide monocarboxylique saturé, obtenus tous deux par hydrolyse dudit ester d'acide saturé, au moins un sous-produit du type éther choisi dans le groupe comprenant l'éther de diméthyle et un éther dérivé dudit alcool organique, et à titre facultatif, du méthylal n'ayant pas réagi et du formaldéhyde dérivé du méthylal;

(c) à séparer et à recueillir ledit sous-produit du type éther du mélange effluent et à recycler au moins une portion dudit sous-produit du type éther en vue de sa réunion avec le mélange de l'étape (a) et de sa réaction conformément à l'étape (b), la quantité dudit éther recyclé étant suffisante pour élever le rapport molaire de l'ester d'acide à no-nsaturation alpha, bêta-éthylénique à l'acide à non-saturation alpha, bêta-éthylénique dans le mélange effluent par rapport à l'absence dudit sous-produit du type éther; ledit mélange effluent, après que le sous-produit du type éther en a été recueilli, étant appelé mélange effluent résiduel;

(d) à recueillir au moins une portion de l'acide monocarboxylique saturé présent dans le mélange effluent résiduel;

(e) à recueillir au moins une portion de l'acide à non-saturation alpha, bêta-éthylénique du mélange effluent résiduel;

(f) à recueillir au moins une portion de l'ester d'acide à non-saturation alpha, bêta-éthylénique et de l'eau du mélange effluent résiduel;

(g) à recueillir au moins une portion de l'ester d'acide monocarboxylique saturé n'ayant pas réagi du mélange effluent résiduel;

(h) à mélanger et à faire réagir dans au moins une zone de réaction séparée de la zone de réaction de l'étape (b) au moins une portion du sous-produit du type éther recueilli conformément à l'étape (c) avec: (i) au moins une portion de l'acide monocarboxylique saturé recueilli conformément à l'étape (d) et (ii) à titre facultatif, au moins une portion de l'acide à non-saturation alpha, bêta-éthylénique recueilli conformément à l'étape (e), d'une manière et dans des conditions suffisantes pour convertir au moins une portion du corps réactionnel (i) en son ester d'acide saturé correspondant et le corps réactionnel (ii), lorsqu'il est présent dans ledit mélange, en son ester d'acide non saturé correspondant, et à recueillir une portion au moins des esters ainsi produits;

(i) à recycler une portion au moins de l'ester d'acide monocarboxylique saturé recueilli conformément à une ou plusieurs des étapes (g) et (h) dans le mélange de l'étape (a) en vue de sa réaction conformément à l'étape (b); et

(j) à titre facultatif, à recueillir une portion au moins du méthylal et du formaldéhyde lorsqu'ils sont présents dans le mélange effluent résiduel et à recycler au moins une portion de ce méthylal et de ce formaldéhyde en vue de leur réunion avec le mélange de l'étape (c) et de leur réaction conformément à l'étape (b).

31. Procédé suivant la revendication 30, dans lequel la totalité de l'ester d'acide monocarboxylique saturé mélangé conformément à l'étape (a) est produite par modification de l'étape (h) de manière qu'elle renferme comme autre corps réactionnel devant réagir ave ledit sous-produit du type éther, de l'acide monocarboxylique saturé frais introduit depuis une source extérieure au mélange effluent résiduel.

32. Procédé suivant la revendication 30, dans lequel ledit ester d'acide monocarboxylique saturé est représenté par la formule structurale

$$R—CH_2—COOR'$$

dans laquelle R et R' sont des groupes hydrocarbyle choisis indépendamment parmi des radicaux alkyle,

aryle, aralkyle et alkaryle et ledit sous-produit du type éther comprend au moins un éther représenté par la formule structurale

R'—O—R'

dans laquelle R' a la définition donnée ci-dessus.

33. Procédé suivant la revendication 32, dans lequel R et R' représentent des groupes alkyle en $C_1$ à $C_5$.

34. Procédé suivant la revendication 30, dans lequel l'ester d'acide monocarboxylique saturé est le propionate de méthyle, l'ester d'acide à non-saturation alpha, bêta-éthylénique correspondant est le méthacrylate de méthyle, et le sous-produit du type éther est l'éther de diméthyle.

35. Procédé suivant la revendication 30, dans lequel une portion au moins de l'eau recueillie conformément à l'étape (f) est recyclée dans la zone de réaction de l'étape (h) d'une manière et dans des conditions suffisantes pour régler la quantité d'éther formé comme sous-produit en mélange avec lesdits corps réactionnels acides par hydrolyse dudit éther.

36. Procédé suivant la revendication 30, dans lequel le catalyseur de déshydratation est acide.

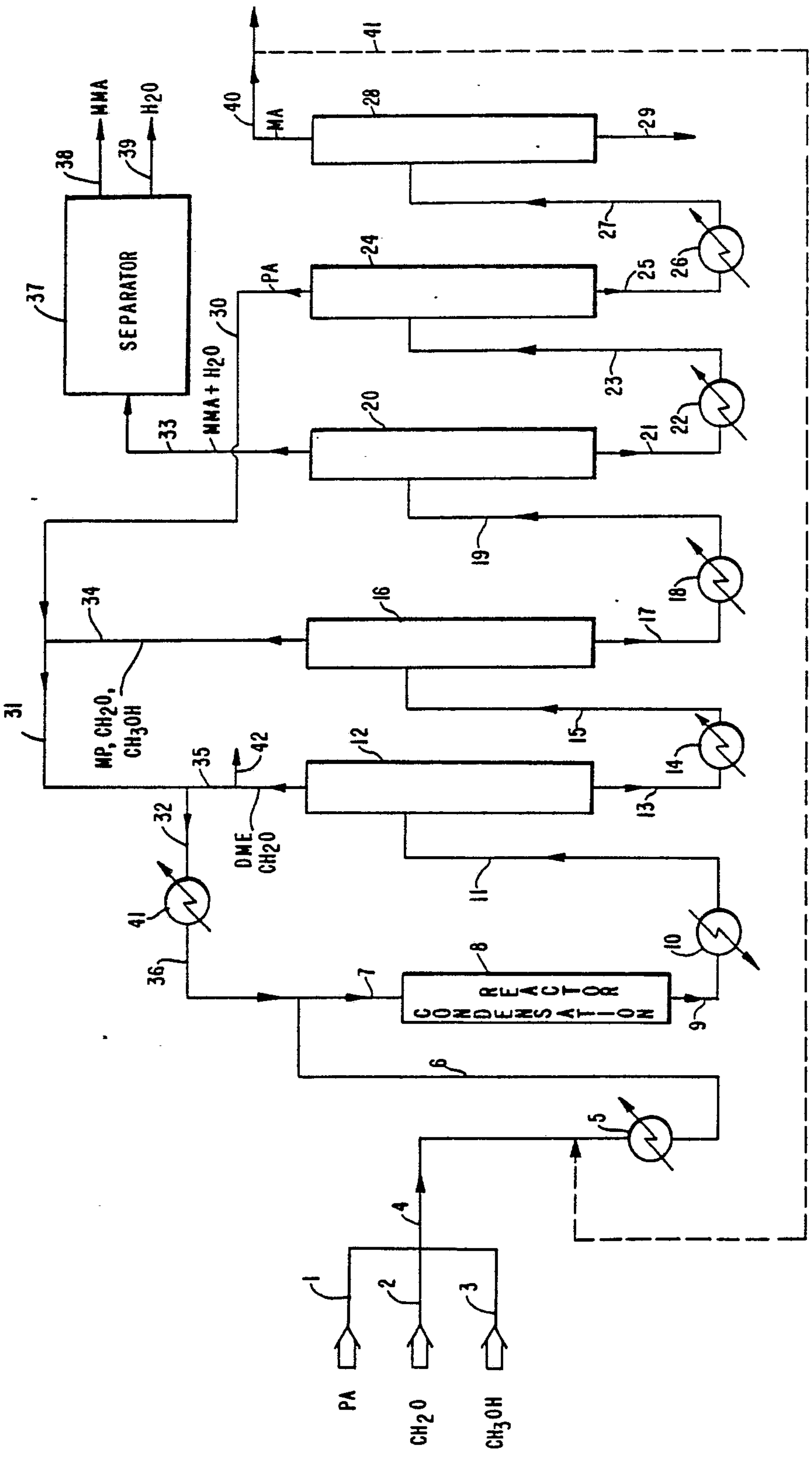
PA
CH2O
CH3OH
CONDENSATION REACTOR
SEPARATOR
MMA
H2O
MA
PA
MMA + H2O
MP, CH2O, CH3OH
DME CH2O

FIG. I

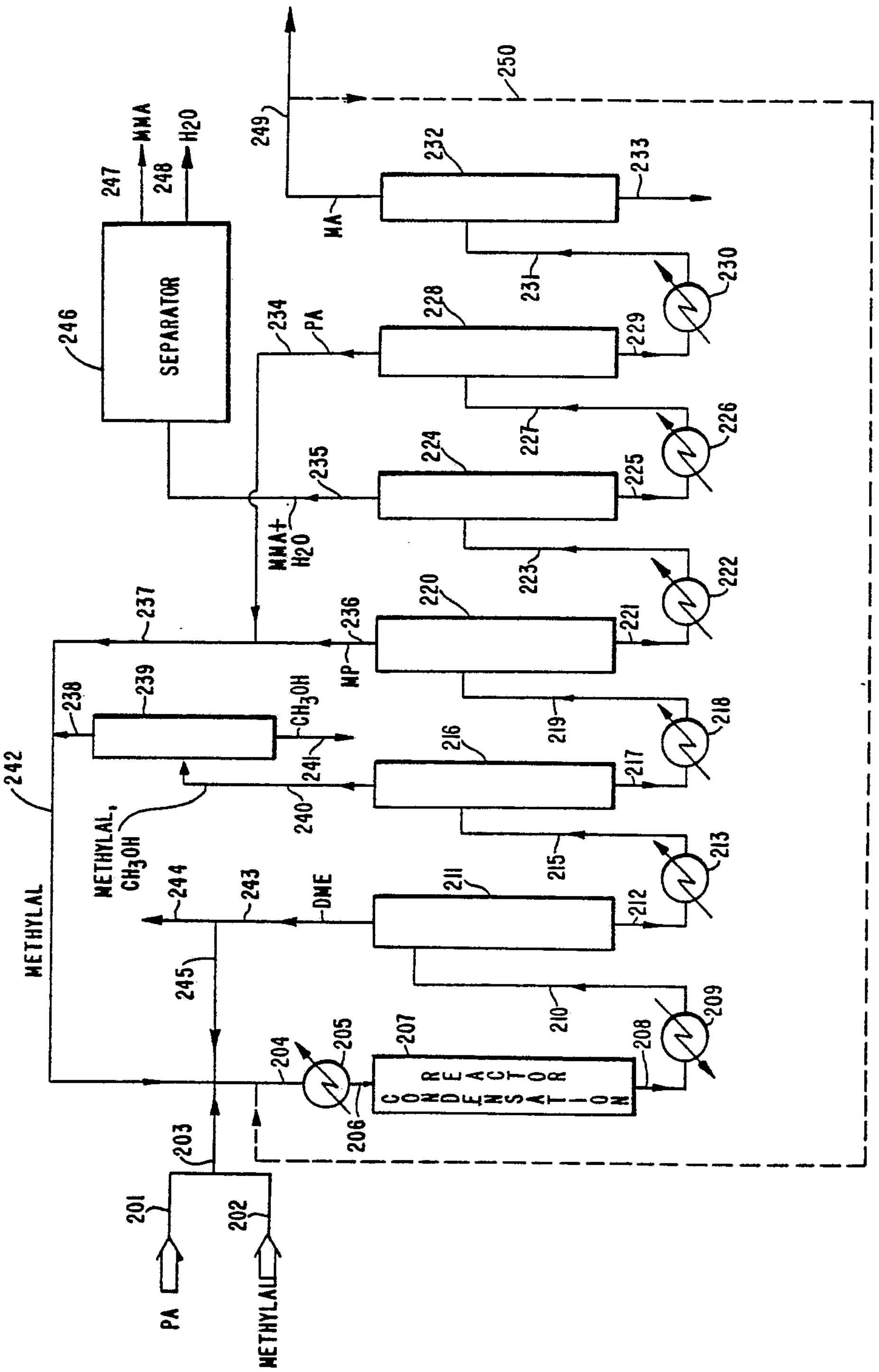
SEPARATOR
MMA
H2O
PA
MA
MMA+H2O
MP
$CH_3OH$
METHYLAL, $CH_3OH$
DME
METHYLAL
CONDENSATION REACTOR
PA
METHYLAL

FIG. 2

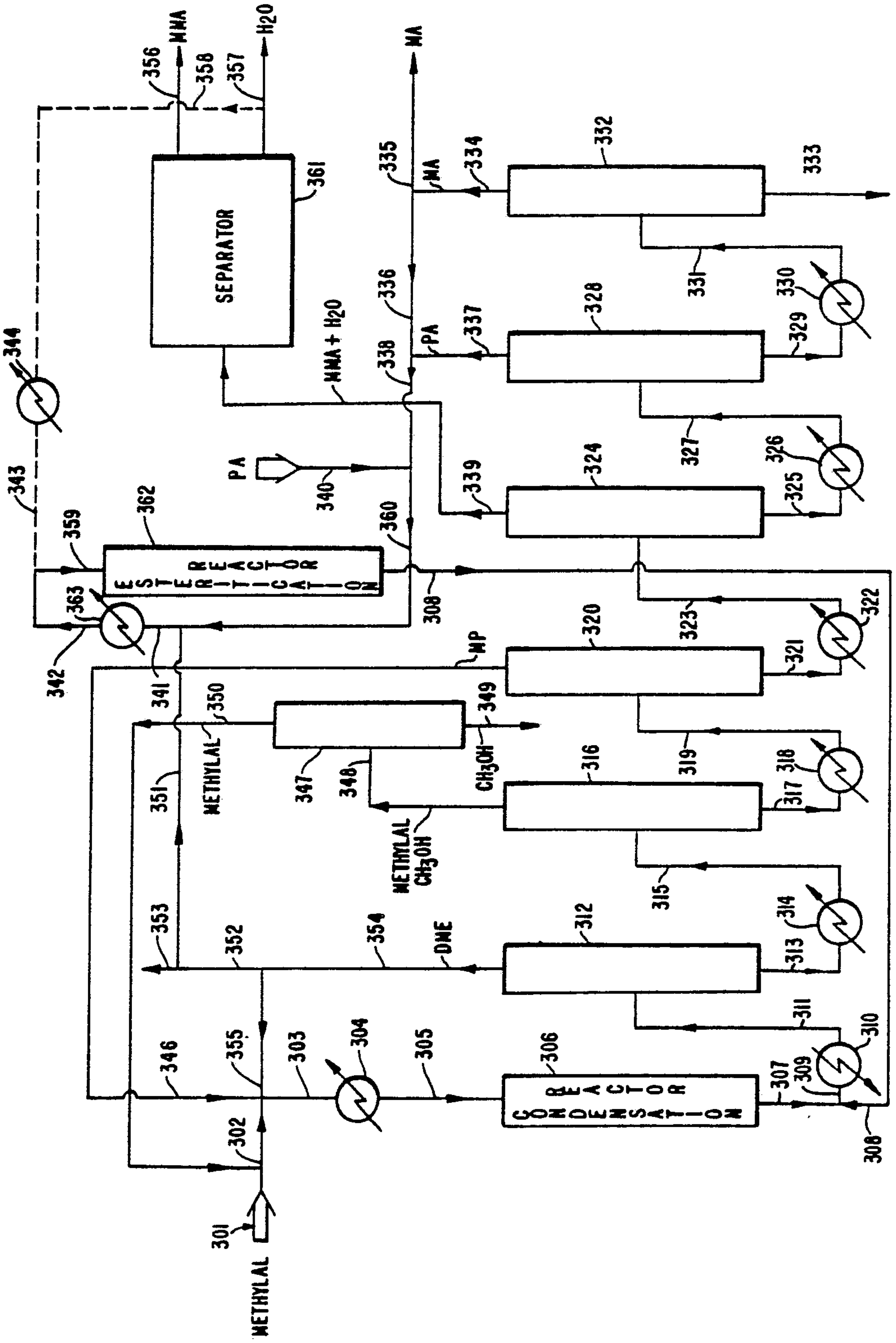
METHYLAL
301
302
303
304
305
306
CONDENSATION REACTOR
307
308
309
310
311
312
313
314
315
316
317
318
319
320
321
322
323
324
325
326
327
328
329
330
331
332
333
334
335
MA
MA
336
337
PA
338
MMA + H2O
339
340
PA
341
342
343
344
346
347
348
METHYLAL CH3OH
349
CH3OH
350
METHYLAL
351
352
353
354
DME
355
356
MMA
357
358
H2O
359
360
361
SEPARATOR
362
ESTERIFICATION REACTOR
363
MP

FIG. 3

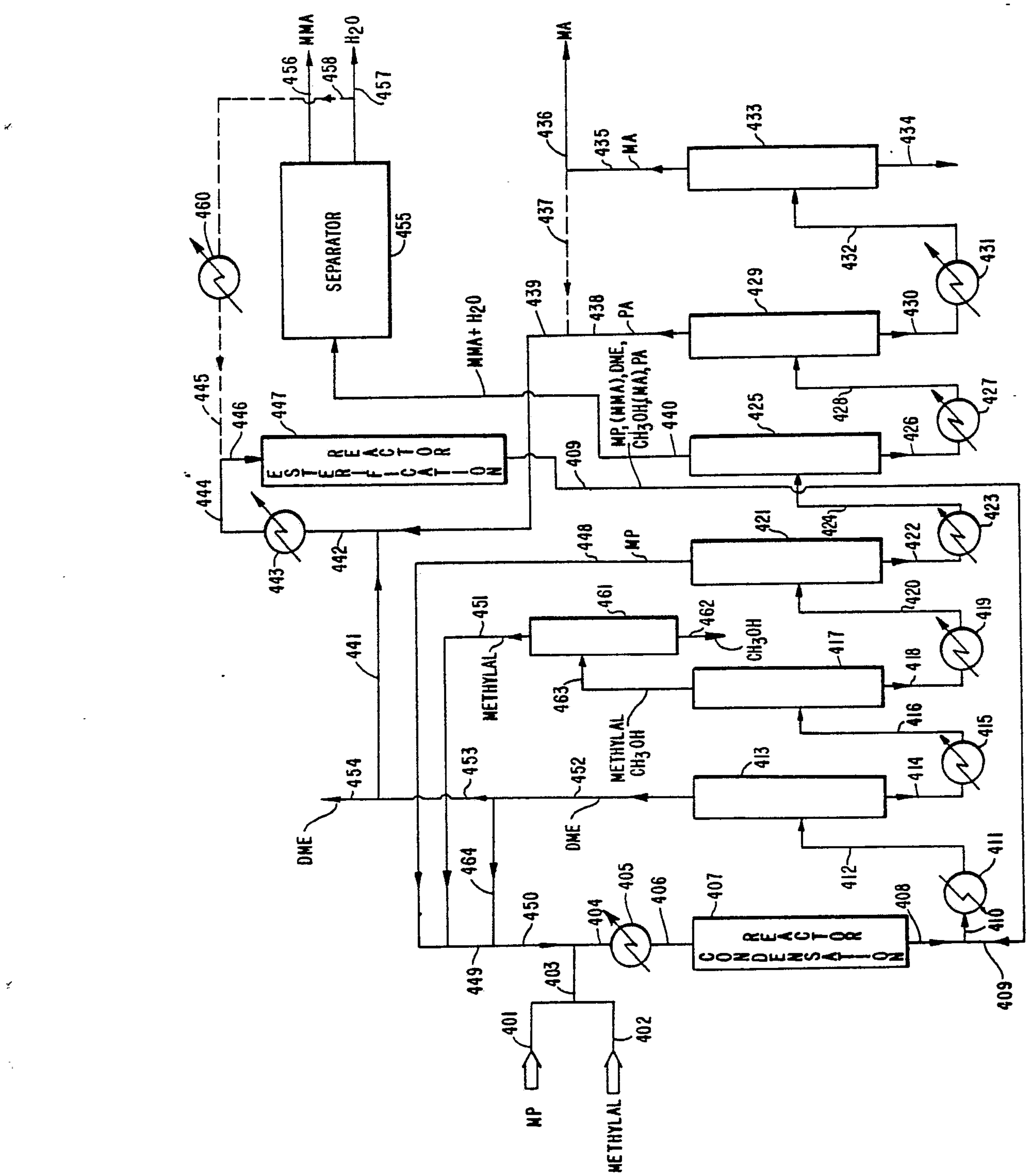
SEPARATOR
ESTERIFICATION REACTOR
CONDENSATION REACTOR
MMA
H2O
MA
PA
MMA+ H2O
MP, (MMA), DME, CH3OH, (MA), PA
MP
METHYLAL
CH3OH
METHYLAL CH3OH
DME


FIG.4